# EUROPEAN PATENT SPECIFICATION

(11) **EP 3 022 313 B1**
(45) Date of publication and mention of the grant of the patent: **15.03.2017**
(21) Application number: 14732874.4
(22) Date of filing: 24.06.2014
(51) Int. Cl.: C12Q 1/10, C12Q 1/68, G01N 33/569, G01N 33/92

(54) **ESCHERICHIA COLI AS A MARKER FOR HYPERTRIGLYCERIDEMIA**
ESCHERICHIA COLI ALS MARKER FÜR HYPERTRIGLYCERIDÄMIE
ESCHERICHIA COLI EN TANT QUE MARQUEUR POUR L'HYPERTRIGLYCÉRIDÉMIE

(30) Priority: 18.07.2013 EP 13176965
(43) Date of publication of application: 25.05.2016
(73) Proprietor: Nestec S.A., 1800 Vevey (CH)
(72) Inventor: CHOU, Chieh Jason, CH-1071 Chexbres (CH); DARIMONT-NICOLAU, Christian, 1012 Lausanne (CH); MEMBREZ, Mathieu, CH-1053 Cugy (CH)
(74) Representative: Kamibayashi, Lynne
(86) International application number: PCT/EP2014/063222
(87) International publication number: WO 2015/007464

(56) References cited:
- EP-A1- 1 757 699
- WO-A1-2008/009869
- WO-A1-2012/131099
- US-A1- 2011 123 501
- GALLIN J I ET AL: "Serum concentrations of lipids in rabbits infected with Escherichia coli and Staphylococcus aureus", PROCEEDINGS OF THE SOCIETY FOR EXPERIMENTAL BIOLOGY & MEDICINE, ACADEMIC PRESS INC. NEW YORK, US, vol. 133, no. 1, 1 January 1970 (1970-01-01), pages 309-313, XP008166286, ISSN: 0037-9727, DOI: 10.3181/00379727-133-34463
- M. MEMBREZ ET AL: "Gut microbiota modulation with norfloxacin and ampicillin enhances glucose tolerance in mice", THE FASEB JOURNAL, vol. 22, no. 7, 1 July 2008 (2008-07-01), pages 2416-2426, XP055093644, ISSN: 0892-6638, DOI: 10.1096/fj.07-102723
- ABU-SHANAB A ET AL: "The role of the gut microbiota in nonalcoholic fatty liver disease", NATURE REVIEWS / GASTROENTEROLOGY & HEPATOLOGY, NATURE, US, vol. 7, no. 12, 1 December 2010 (2010-12-01), pages 691-701, XP008166282, ISSN: 1759-5045, DOI: 10.1038/NRGASTRO.2010.172

### SEQUENCE LISTING

<110> Chou, Chieh Jason
   Darimont, Christian
   Membrez, Mathieu
<120> E.coli As a Marker For
   Hypertriglyceridemia
<130> 13102-PCT
<160> 31
<170> FastSEQ for Windows Version 4.0
<210> 1
   <211> 19
   <212> DNA
   <213> Artificial Sequence
<220>
   <223> synthetic PCR forward primer for detecting total bacteria
<400> 1
   tcctacggga ggcagcagt 19
<210> 2
   <211> 26
   <212> DNA
   <213> Artificial Sequence
<220>
   <223> synthetic PCR reverse primer for detecting total bacteria
<400> 2
   ggactaccag ggtatctaat cctgtt 26
<210> 3
   <211> 20
   <212> DNA
   <213> Artificial Sequence
<220>
   <223> synthetic PCR forward primer for detecting Escherichia coli
<400> 3
   catgccgcgt gtatgaagaa 20
<210> 4
   <211> 21
   <212> DNA
   <213> Artificial Sequence
<220>
   <223> synthetic PCR reverse primer for detecting Escherichia coli
<400> 4
   cgggtaacgt caatgagcaa a 21
<210> 5
   <211> 25
   <212> DNA
   <213> Artificial Sequence
<220>
   <223> synthetic PCR forward primer for detecting Firmicutes
<400> 5
   ggagyatgtg gtttaattcg aagca 25
<210> 6
   <211> 20
   <212> DNA
   <213> Artificial Sequence
<220>
   <223> synthetic PCR reverse primer for detecting Firmicutes
<400> 6
   agctgacgac aaccatgcac 20
<210> 7
   <211> 19
   <212> DNA
   <213> Artificial Sequence
<220>
   <223> synthetic PCR forward primer for detecting Lactobacillus
<400> 7
   agcagtaggg aatcttcca 19
<210> 8
   <211> 17
   <212> DNA
   <213> Artificial Sequence
<220>
   <223> synthetic PCR reverse primer for detecting Lactobacillus
<400> 8
   caccgctaca catggag 17
<210> 9
   <211> 25
   <212> DNA
   <213> Artificial Sequence
<220>
   <223> synthetic PCR forward primer for detecting Bacteroidetes
<400> 9
   ggarcatgtg gtttaattcg atgat 25
<210> 10
   <211> 20
   <212> DNA
   <213> Artificial Sequence
<220>
   <223> synthetic PCR reverse primer for detecting Bacteroidetes
<400> 10
   agctgacgac aaccatgcag 20
<210> 11
   <211> 20
   <212> DNA
   <213> Artificial Sequence
<220>
   <223> synthetic PCR forward primer for detecting Bacteroidaceae-Prevotella-Porphyromonas
<400> 11
   ggtgtcggct taagtgccat 20
<210> 12
   <211> 18
   <212> DNA
   <213> Artificial Sequence
<220>
   <223> synthetic PCR reverse primer for detecting Bacteroidaceae-Prevotella-Porphyromonas
<400> 12
   cggaygtaag ggccgtgc 18
<210> 13
   <211> 17
   <212> DNA
   <213> Artificial Sequence
<220>
   <223> synthetic PCR forward primer for detecting Bifidobacterium
<400> 13
   ctcctggaaa cgggtgg 17
<210> 14
   <211> 22
   <212> DNA
   <213> Artificial Sequence
<220>
   <223> synthetic PCR reverse primer for detecting Bifidobacterium
<400> 14
   ggtgttcttc ccgatatcta ca 22
<210> 15
   <211> 23
   <212> DNA
   <213> Artificial Sequence
<220>
   <223> synthetic PCR forward primer for detecting Enterobacteriaceae
<400> 15
   tgccgtaact tcgggagaag gca 23
<210> 16
   <211> 22
   <212> DNA
   <213> Artificial Sequence
<220>
   <223> synthetic PCR reverse primer for detecting Enterobacteriaceae
<400> 16
   tcaaggctca atgttcagtg tc 22
<210> 17
   <211> 18
   <212> DNA
   <213> Artificial Sequence
<220>
   <223> synthetic RT-PCR forward primer for detecting NFkB mRNA expression
<400> 17
   cggacagccc tccacctt 18
<210> 18
   <211> 21
   <212> DNA
   <213> Artificial Sequence
<220>
   <223> synthetic RT-PCR reverse primer for detecting NFkB mRNA expression
<400> 18
   cagccccaca tttcaacaag a 21
<210> 19
   <211> 24
   <212> DNA
   <213> Artificial Sequence
<220>
   <223> synthetic RT-PCR forward primer for detecting IL-6 mRNA expression
<400> 19
   gaaatgatgg atgctaccaa actg 24
<210> 20
   <211> 24
   <212> DNA
   <213> Artificial Sequence
<220>
   <223> synthetic RT-PCR reverse primer for detecting IL-6 mRNA expression
<400> 20
   ccagaagacc agaggaaatt ttca 24
<210> 21
   <211> 20
   <212> DNA
   <213> Artificial Sequence
<220>
   <223> synthetic RT-PCR forward primer for detecting TNF-alpha mRNA expression
<400> 21
   accgtcagcc gatttgctat 20
<210> 22
   <211> 20
   <212> DNA
   <213> Artificial Sequence
<220>
   <223> synthetic RT-PCR reverse primer for detecting TNF-alpha mRNA expression
<400> 22
   ttgacggcag agaggaggtt 20
<210> 23
   <211> 20
   <212> DNA
   <213> Artificial Sequence
<220>
   <223> synthetic RT-PCR forward primer for detecting ACC2 mRNA expression
<400> 23
   agcgggactc tgtcctcaag 20
<210> 24
   <211> 23
   <212> DNA
   <213> Artificial Sequence
<220>
   <223> synthetic RT-PCR reverse primer for detecting ACC2 mRNA expression
<400> 24
   ccatgatgac ctctggatgt tct 23
<210> 25
   <211> 22
   <212> DNA
   <213> Artificial Sequence
<220>
   <223> synthetic RT-PCR forward primer for detecting NFkB mRNA expression
<400> 25
   accatccaga tgacaccttc ct 22
<210> 26
   <211> 20
   <212> DNA
   <213> Artificial Sequence
<220>
   <223> synthetic RT-PCR reverse primer for detecting NFkB mRNA expression
<400> 26
   gccgaaggtc caccattttt 20
<210> 27
   <211> 21
   <212> DNA
   <213> Artificial Sequence
<220>
   <223> synthetic RT-PCR forward primer for detecting MTTP mRNA expression
<400> 27
   cacctcagcc agaaagcgat a 21
<210> 28
   <211> 18
   <212> DNA
   <213> Artificial Sequence
<220>
   <223> synthetic RT-PCR reverse primer for detecting MTTP mRNA expression
<400> 28
   agggaaccca cgggaatc 18
<210> 29
   <211> 22
   <212> DNA
   <213> Artificial Sequence
<220>
   <223> synthetic ERIC-PCR primer
<400> 29
   atgtaagctc ctggggattc ac 22
<210> 30
   <211> 22
   <212> DNA
   <213> Artificial Sequence
<220>
   <223> synthetic ERIC-PCR primer
<400> 30
   aagtaagtga ctggggtgag cg 22
<210> 31
   <211> 22
   <212> DNA
   <213> Artificial Sequence
<220>
   <223> synthetic rep-PCR primer
<400> 31
   ctacggcaag gcgacgctga cg 22

## Description

### BACKGROUND OF THE INVENTION

Dyslipidemia is a disorder of lipoprotein metabolism that is characterized by an abnormal amount of lipids (*e*.*g*., cholesterol and/or fat) present in the blood. The prevalence of dyslipidemias, particularly hyperlipidemias, such as hypertriglyceridemia (HTG), hypercholesterolemia, hyperlipoproteinemia, or a combination thereof, is high in developing countries. For example, in the United States the prevalence of hypertriglyceridemia, defined as plasma triglyceride level above 1.2 mmol/liter (150 mg/dl), is about 27.6% for women and 35.1% for men (Mozumdar and Liguori, Diabetes Care, 34(1):216-219 (2011); Ford et al, JAMA, 287(3):356-359 (2002)).

HTG can result from increased triglyceride production, reduced triglyceride catabolism, or even both. Classic risk factors of hypertriglyceridemia include consumption of fatty food, excess alcohol intake, sedentary lifestyle, age, obesity, and/or the presence of metabolic disease. Other risk factors include type 2 diabetes, genetic disorders (*e*.*g*., familial hypertriglyceridemia, familial combined hyperlipidemia, familial dysbetalipoproteinemia), certain drugs (*e*.*g*., steroids, beta-blockers, and high-estrogen oral contraceptives), and other diseases (*e*.*g*., hypothyroidism, hyperhomocystinemia, Cushing's disease, other endocrine, renal or liver diseases, and autoimmune disorders).

Typically, HTG is diagnosed according to discrete categories based on fasting plasma triglyceride (TG) levels. For instance, the Endocrine Society's classification system defines that normal TG is <1.7mmol/liter (<150 mg/dl, mild HTG is 1.7-2.3 mmol/liter (150-199 mg/dl), moderate HTG is 2.3-11.2 mmol/liter (200-900 mg/dl), severe HTG is 11.2-22.4 mmol/liter (1000-1999 mg/dl), and very severe HTG is >22.4 mmol/liter (>2000 mg/dl).

It has been shown the gut microbiota plays a role in metabolic disorders such as obesity, diabetes, and cardiovascular diseases. Without being bound by any particular theory, it is believed that gut bacteria can induce an innate immune response in the host individual by way of lipopolysaccharides (LPS) found on the bacterial cell wall, resulting in host inflammation and a cascade of other physiological events. Additionally, it is thought that bacterial metabolites of dietary compounds can regulate biological activities in the host, thereby contributing to metabolic disorders.

WO2008/009869 describes the correlation of high bacterial endotoxin (LPS) levels and metabolic disorders, i. e. dyslipidemia of the high triglyceride/low HDL cholesterol type, in serum or blood samples.

Classic markers for HTG lack specificity to address an individual's risk level of acquiring the disorder. Unfortunately, rapid and accurate diagnostic methods for predicting an individual's risk of developing metabolic hypertriglyceridemia are not currently available. The present invention satisfies this need and provides related advantages as well.

### BRIEF SUMMARY OF THE INVENTION

The present invention is in its broadest sense defined by the appended claims. In one embodiment, the present invention provides a method for predicting whether a subject is at risk of developing hypertriglyceridemia. The method comprises the steps of: a) transforming a stool sample obtained from the subject to detect the level of *Escherichia coli* (*E. coli*) in the sample; and b) determining that a higher level *of E. coli* in the sample compared to a control sample predicts that the subject is at risk of developing hypertriglyceridemia.

In some aspects, detecting the level of *E*. *coli* comprises performing nucleic acid amplification. In other aspects, detecting the level of *E*. *coli* comprises colony counting.

In some aspects, the control sample is from a healthy control subject or a subject that is not at risk of developing hypertriglyceridemia.

In yet another embodiment, the invention provides a method for diagnosing hypertriglyceridemia in a subject, wherein the method comprises a) transforming a stool sample obtained from the subject to detect the level of *E. coli* in the sample; and b) determining that a higher level of *E. coli* in the sample compared to a control sample indicates a diagnosis of hypertriglyceridemia in the subject.

In some aspects, detecting the level *of E. coli* comprises performing nucleic acid amplification. In some aspects, nucleic acid amplification is performed using oligonucleotides whose sequences are set forth in SEQ ID NOs:3 and 4. In other embodiments, nucleic acid amplification comprises enterobacterial repetitive intergenic consensus PCR or repetitive extragenic PCR.

Other objects, features, and advantages of the present invention will be more apparent to one of skill in the art from the following detailed description.

### DETAILED DESCRIPTION OF THE INVENTION

### I. Introduction

Hypertriglyceridemia (HTG) is a condition wherein fasting serum triglyceride (TG) levels are elevated due to overproduction and/or impaired removal of triglycerides or triglyceride-rich lipoprotein particles. It can be caused by genetic defects that lead to disordered TG metabolism. Or, it can be caused by other lipid and metabolic conditions, including high fat diet, excessive alcohol intake, certain medications, diabetes mellitus, hypothyroidism, insulin resistance, and hypertension.

Predicting an individual's risk of developing HTG can be challenging due to the lack of specificity of currently available biomarkers. Furthermore, it has been recommended that the presence of elevated triglyceride levels should always be evaluated in the context of other risk factors for metabolic disease and cardiovascular disease, including secondary causes of hyperlipidemia, family history of dyslipidemia and cardiovascular disease, obesity, hypertension, glucose metabolism abnormalities, liver dysfunction, *etc.*

It has been discovered that an elevated level *of E. coli* in the gastrointestinal system of an individual is predictive of developing hypertriglyceridemia. The present invention provides methods for assessing an individual's risk of acquiring hypertriglyceridemia, as well as providing an effective therapy for treating the disorder.

In certain aspects, practicing this invention utilizes techniques in the field of molecular biology. Basic texts disclosing the general methods of use in this invention include Sambrook and Russell, Molecular Closing, A Laboratory Manual (3rd ed. 2001); Kriegler, Gene Transfer and Expression: A Laboratory Manual (1990); and Current Protocols in Molecular Biology (Ausubel et al., eds., 1994).

### II. Definitions

As used herein, the following terms have the meanings ascribed to them unless specified otherwise.

"Dyslipidemia" includes any condition defined as an elevation of plasma cholesterol, triglycerides (TGs), or both, or a low high-density lipoprotein level. Dyslipidemia can contribute to the development of atherosclerosis, and thereby increases the risk of atherosclerosis. There are different classes of dyslipidemia that are distinguished by the patterns of elevated lipids and lipoprotein. Class I is defined as elevated chylomicrons and TG, class IIa as elevated low-density lipoprotein (LDL) and cholesterol, class IIb as elevated very-low-density lipoprotein (VLDL) and LDL and elevated TG and cholesterol, class III- as elevated VLDL and chylomicron remnants and elevated TG and cholesterol, class IV as elevated VLDL and TGs, and class V as elevated chlylomicrons and VLDL and elevated TGs and cholesterol.

The term "hypertriglyceridemia" includes a fasting plasma triglyceride (TG) measurement that is above a certain cut-off threshold point or is increased or elevated compared to an age- and sex-matched healthy control. The diagnosis can be defined as when plasma TG concentration rises above a threshold value, such as the 90^{th} or 95^{th} percentile for an individual's age and sex.

The two main sources of plasma triglycerides are exogenous (from dietary fats) and carried in chylomicrons, and endogenous (from the liver) and carried in very-low-density lipoprotein (VLDL) particles.

The term "sample" includes any biological specimen obtained from an individual. Samples include whole blood, plasma, serum, saliva, urine, stool, tears, any other bodily fluid, tissue samples (e.g., biopsy), and cellular extracts thereof (*e.g_{.}*, red blood cellular extract). In the present invention the sample is a stool sample.

The term "individual," "subject," or "patient" typically includes humans, but also includes other animals such as, *e.g.,* other primates, rodents, canines, felines, equines, ovines, porcines, and the like.

The term "transforming the sample" includes a physical or chemical change of the sample to extract and /or obtain a marker *(e.g., E. coli* or other bacteria) as defined herein. An extraction, a manipulation, a chemical precipitation, an ELISA, an immuno-extraction, a physical or chemical modification of the sample to measure a marker all constitute a transformation. As long as the sample is not identical before and after the transformation step, the change is a transformation.

The term "classifying" includes "to associate" or "to categorize" a sample with a disease state. In certain instances, "classifying" is based on statistical evidence, empirical evidence, or both. In certain embodiments, the methods and systems of classifying use a so-called training set of samples having known disease states. Once established, the training data set serves as a basis, model, or template against which the features of an unknown sample are compared, in order to classify the unknown disease state of the sample. In certain instances, classifying the sample is akin to diagnosing the disease state of the sample. In certain other instances, classifying the sample is akin to differentiating the disease state of the sample from another disease state.

The term "dietary intervention" includes a treatment related to an individual's diet or dietary intake. The treatment includes diet related components, restrictions, recommendations directed to preventing a deficiency, treating a disease or medical condition in an individual or, more generally, reducing symptoms, managing progression of the disease or providing a nutritional, physiological, or medical benefit to the individual. Examples of dietary interventions include, but are not limited to, reduction of carbohydrates, reduction of fats, weight control, reduction of alcohol consumption, increasing physical activity and maintaining a low-fat or very-low-fat diet.

The term "nutritional intervention" includes a treatment related to an individual's nutritional intake. The treatment includes nutrition related components, supplements, recommendations directed to preventing a deficiency, treating a disease or medical condition in an individual or, more generally, reducing symptoms, managing progression of the disease or providing a nutritional, physiological, or medical benefit to the individual. Examples of nutritional interventions include, but are not limited to, omega-3 fatty acid *(e.g.,* fish oil), phytosterols, long chain polyunsaturated fatty acids (LC-PUFA), taurine, probiotics (e.g., *Lactobacillus plantarum, Bifidobacterium longum,* etc.), carbohydrates (*e.g.,* resistant starch, β-cyclodextran, etc.), proteins (*e.g.,* soy protein, β-conglycinin, glycinin, etc.), dietary fiber (*e.g.,* psyllium, pectin, β-glucan, fructo-oligosaccharide, inulin, etc.), phytonutrients (*e*.*g*., isoflavone, grape polyphenols, resveratrol, punicalagin in pomegranate, green tea, oolong tea, etc.), eicosanoid, palmitoleic acid, and combinations thereof.

The term "therapeutically effective amount or dose" includes a dose of a drug that is capable of achieving a therapeutic effect in a subject in need thereof. For example, a therapeutically effective amount of a drug useful for treating hypertriglyceridemia can be the amount that is capable of preventing or relieving one or more symptoms associated with hypertriglyceridemia. The exact amount can be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

### III. Detailed Description of the Embodiments

Provided herein is a method for predicting hypertriglyceridemia (HTG) in a patient by measuring the level *of E. coli* (*e.g.,* enteric *E. coli*) in a sample, i.e. a stool sample from the patient. Also described herein is a a method for treating a patient at risk of or susceptible to developing HTG. The present invention thus provides an accurate diagnostic prediction of HTC that is useful for guiding treatment decisions.

### A. Detecting E. coli

### 1. Preparation of samples

The method described herein relates to measuring the amount *of E. coli* or the amount of DNA or RNA specific to *E. coli* found in an individual's gastrointestinal tissue, i.e. a stool sample, as a means to predict whether a subject is at risk of developing or to diagnose hypertriglyceridemia. From a stool sample obtained from an individual nucleic acids are extracted.

In some embodiments, an appropriate amount of a sample is collected and may be stored according to standard procedures prior to further preparation.

In some instances, the sample is suspended in a buffer or culture medium and then plated at various dilutions on either a semi-selective or selective culture medium (e.g., Drigalski growth medium) for enrichment of bacteria.

The analysis of *E. coli* RNA or DNA found in an individual's sample according to the present invention may be performed using stool. The methods for preparing samples for nucleic acid extraction are well known among those of skill in the art. For example, a subject's sample should be first treated to disrupt cellular membrane so as to release nucleic acids contained within the cells.

### 2. Extraction and quantitation of nucleic acid

Methods for extracting RNA or DNA from a biological sample are well known and routinely practiced in the art of molecular biology, see, *e.g.,* Sambrook and Russell, *supra.* RNA contamination should be eliminated to avoid interference with DNA analysis. Commercially available kits, e.g., QIAamp DNA stool mini kit (Qiagen, Germantown, MD), UltraClean Fecal DNA Isolation Kit (MO BIO Laboratories, Carlsbad, CA), MoBio PowerSoil® DNA Isolation kit (MO BIO Laboratories, Carlsbad, CA), PowerMicrobiome™ RNA Isolation Kit (MO BIO Laboratories, Carlsbad, CA), Stool Nucleic Acid Isolation Kit (Norgen Biotek Corp., Ontario Canada) can also be used for extraction of bacterial nucleic acids.

### 3. Nucleic acid detection and quantitation

Assays that can be used to determine the level (amount) *of E. coli* DNA or RNA (and thereby the level of *E. coli* bacteria) in a sample include, but are not limited to, electrophoretic analysis, restriction length polymorphism analysis, sequence analysis, hybridization analysis, ribotyping analysis, ribosomal DNA heterogeneity analysis, and PCR analysis, including quantitative PCR (qPCR), quantitative real-time PCR (qRT-PCR), PCR-based denaturing-gradient-gel-electrophoresis (PCR-DGGE), repetitive extragenic palindromic-PCR (rep-PCR) and enterobacterial repetitive intergenic consensus PCR (ERIC-PCR). These assays have been well-described and standard methods are known in the art. *See, e.g.,* Ausubel et al., Current Protocols in Molecular Biology, John Wiley & Sons, Inc. New York (1984-2008), Chapter 7 and Supplement 47; Theophilus et al., "PCR Mutation Detection Protocols," Humana Press, (2002); Innis et al., PCR Protocols, San Diego, Academic Press, Inc. (1990); Maniatis et al., Molecular Cloning: A Laboratory Manual, Cold Spring Harbor Lab., New York, (1982); Ausubel et al., Current Protocols in Genetics and Genomics, John Wiley & Sons, Inc. New York (1984-2008); and Ausubel et al., Current Protocols in Human Genetics, John Wiley & Sons, Inc. New York (1994-2008).

In some embodiments, the *E.coli* level in a sample is quantified by standard nucleic acid amplification methods such as PCR analysis. In one embodiment, the following oligonucleotide primer pair is used: 5'-CATGCCGCGTGTATGAAGAA (SEQ ID NO:3) and 5'-CGGGTAACGTCAATGAGCAAA (SEQ ID NO:4). The reaction condition can include the following steps: denaturation at 50°C for 2 min, PCR amplification as follows: 95°C for 10 min, 95°C for 15 sec, 60°C for 1 min for 40 cycles. The amplification product can be detected and measured by standard methods known in the art.

In another embodiment, 16S rRNA gene specific PCR is used to detect *E. coli* levels. For instance, hypervariable regions of bacterial 16S rRNA can be amplified with region-specific primers to generate amplicons.

ERIC-PCR refers to a type of PCR analysis wherein an amplification product is generated with primers for specific enterobacterial repetitive sequences *(see, e.g.,* Dalla-Costa et al., J. Med. Microbiol., 47:227-234 (1998). In one embodiment of the present invention, the level of E. *coli* DNA in a sample is determined by performing ERIC-PCR with the following oligonucleotide primer pairs: 5'-ATGTAAGCTCCTGGGGATTCAC (SEQ ID NO:29) and 5'-AAGTAAGTGACTGGGGTGAGCG (SEQ ID NO:30). An exemplary PCR reaction mixture can include template DNA, oligonucleotide primers, dNTPs, amplification buffer and Taq DNA polymerase. An exemplary reaction condition includes denaturation at 95°C for 5 min, PCR amplification as follows: 95°C for 45 sec, 52°C for 1 min, 70°C for 10 min for 35 cycles, and extension at 70°C for 20 min. The amplification products can be separated by gel electrophoresis or by other methods known in the art.

Rep-PCR DNA fingerprinting refers to a type of PCR analysis wherein amplification of DNA between adjacent repetitive entragenic elements is used to obtain strain-specific DNA fingerprints. In one embodiment of the present invention, the presence *of E. coli* DNA in a sample is determined by performing rep-PCR with the following oligonucleotide primer: 5'-CTACGGCAAGGCGACGCTGACG (SEQ ID NO:31). In some instances, a whole cell suspension is used as an amplification template. In some embodiments, the reaction condition includes denaturation at 95°C for 2 min, PCR amplification as follows: 95°C for 3 sec, 92°C for 30 sec, 50 °C for 1 min, 65°C for 8 min for 30 cycles, and extension at 65°C for 8 min. The amplification products can be analyzed by gel electrophoresis. Gel images of the amplification products can be digitally imaged and compared to standardized DNA fingerprint images by performing a statistical analysis.

### 4. Bacterial detection and quantitation

In some aspects of the invention, methods such as an immunoassay can be used to detect the level of *E.coli* in a sample. Non-limiting examples of an immunoassay include an ELISA, solid phase immunoassay, Western blot and protein microarray.

In some aspects of the invention, culture-based analysis of bacteria is determined by culturing (plating) bacteria on medium in either aerobic or anaerobic conditions and quantitating the number of colonies formed. In one embodiment, the sample is diluted in medium (*e*.*g*., Ringer medium) and plated onto non-selective media such as TSS medium for about 24-48 hours at 37 °C in aerobic or anaerobic conditions. The number of bacteria in the sample is proportional to the number of colonies formed relative to the size of the sample. A detailed description of the method is found in, *e.g.,* Schumann et al., Physiol. Genomics, 23:235-245 (2005).

*Enterobacteriaceae* in a sample is quantitated by the following method: 1) diluting the sample in medium (*e.g.,* Ringer medium), shaking, and centrifuging *(e.g.,* at 10,000 rpm for 10 min); 2) resuspending the pellet in medium (*e*.*g*., Ringer medium); 3) spreading the cells onto Drigalski and EMB plates and incubating at 37 °C for about 18-24 hours in aerobic conditions; and 4) quantitating the colonies formed. A detailed description of the method is found in, *e.g.,* Membrez et al., FASEB Jour., 22:2416-2426 (2008).

In yet another embodiment, after washing the bacterial cell pellet of step 1) described above, the cell suspension is inoculated into *Enterobacteriaceae* enrichment broth (E.E. broth; Oxoid LTD, Basingstoke, England) for 24 hours at 37 °C, and then the cells are spread on to EMB agar. *Enterobacteriaceae* of the colonies can be identified using standard techniques such as an API ID32E test (Biomerieux, Durham, NC). A detailed description of the method is found in, *e.g.,* Murray et al., Manual of Clinical Microbiology, 9th Ed., American Society for Microbiology, Washington, D.C. (2003).

In some embodiments, bacteria associated with hypertriglyceridemia can be detected by inoculating the sample on a detection medium wherein the media comprises particular substrates, specific for a metabolic activity, such as an enzymatic activity, of the bacterium that it is desired to detect; through the choice of substrates, depending on whether or not there is a reaction, it is possible to characterize the nature of a microorganism. Examples of such methods are described in detail in, *e.g.,* U.S. Patent No. 8,334,112.

The level of *Enterobacteriaceae* can be detected using a binding molecule (*e.g*., antibody, antibody fragment, aptamer, *etc.*) specific to the *Enterobacteriaceae* of interest, i.e. *E.coli.*

In other embodiments, the level of *Enterobacteriaceae* can be measured by detecting a molecule (*e.g*., peptide, protein, enzyme, chemical compound, *etc*.) specific to the *Enterobacteriaceae* of interest, i.e. *E.coli.* In one embodiment, the method for detecting the level of *Enterobacteriaceae,* i.e. *E. coli* in a sample, comprising the steps of contacting the sample with a detectably labeled substrate for an enzyme produced and/or secreted by the bacterium, under conditions that result in modification of the substrate by the enzyme; and detecting the modification or the absence of the modification of the substrate. Modification of the substrate indicates the presence of the *Enterobacteriaceae* in the sample, and the absence of modification of the substrate indicates the absence of the bacterium in the sample. Examples of such methods are described in detail in, *e.g.,* U.S. Patent No. 8,377,651.

Methods for identifying a particular *E. coli* strain can be useful in the invention. Such methods include ribotyping, sequencing (e.g., 16S rRNA sequencing), denaturing gradient gel electrophoresis, terminal restriction fragment length polymorphisms, ribosomal intergenic spacer PCR analysis, DNA microarray, FISH, qPCR, and qRT-PCR.

### B. Predicting hypertriglyceridemia

In some embodiments, if the level *of E.coli* in the individual's sample is higher than that of the control sample (e.g., sample from a healthy control or an individual who does not have hypertriglyceridemia), then it is determined that the individual is at risk or is susceptible to developing HTG. In some embodiment, HTG is predicted if the *E. coli* level is at least about 2 times (2x) higher, *e.g.,* at least about 2x, 3, 4, 5, 6, 7, 8, 9, 10 or more, than that of the control. In some embodiment, HTG is predicted if the *E. coli* level is at least about 5 times (5x) higher, *e.g.,* at least about 5x, 6,x, 7x, 8x, 9x, 10x, 11x, 12x, 13x, 14x, 15x or more, than that of the control.

In some embodiments, if the *E. coli* level in the sample is equal to or lower than that of the control sample, then the individual is not at risk of developing HTG or does not have HTG.

In some embodiments, if the level of *E. coli* in the individual's sample is higher than that of the control sample (*e*.*g*., sample from a healthy control or an individual who does not have hypertriglyceridemia), then the individual is diagnosed as having HTG. In some instances, HTG is diagnosed if the *E. coli* level in the test sample is at least about 2 times (2x) higher, *e.g.,* at least about 2x, 3, 4, 5, 6, 7, 8, 9, 10 or more, than that of the control. In some embodiment, HTG is diagnosed if the *E. coli* level is at least about 5 times (5x) higher, *e.g.,* at least about 5x, 6,x, 7x, 8x, 9x, 10x, 11x, 12x, 13x, 14x, 15x or more, than that of the control.

### C. Therapeutic drug administration

As described herein, the method includes administering a therapeutically effective amount of a therapeutic drug, an antibiotic, a dietary intervention, a nutritional intervention, or combinations thereof, thereby treating hypertriglyceridemia or the symptoms thereof. In some instances, a therapeutically effective amount is selected to reduce the total amount of enterobacteria, or the relative amount of enterobacteria in the gut.

As described herein, the relative amount of enterobacteria to the total amount of bacteria in the gut is reduced or eliminated by about 1% to about 100%, preferably about 50% to about 100%, even more preferably by about 75% to about 100%. Also described herein, the relative amount is reductedby at least about 50%, 60%, 65%, 70%, 75%, 80%, 85%, 90%, 95% or 100%. In some instances, the percentages are calculated based on the colony forming units (cfu) of the bacterial species present in the gut.

As described herein, the therapeutically effective amount of the therapeutic drug is administered alone. As described herein, the therapeutic drug is administered in combination with therapeutically effective amount of a nutritional intervention.

Suitable drugs that are useful for treating one or more symptoms associated with HTG or a clinical subtype thereof include, but are not limited to, a fibrate, including bezafibrate (Bezalip), ciprofibrate (Modalim), clofibrate, gemfibrozil (Lopid), fenofibrate (TriCor), and combinations thereof.

For therapeutic drug applications, the hypertriglyceridemia (HTG) drug can be administered alone or co-administered in combination with one or more additional HTG drugs and/or one or more drugs that reduce the side-effects associated with the HTG drug.

HTG drug(s) can be administered with a suitable pharmaceutical excipient as necessary and can be carried out via any of the accepted modes of administration. Thus, administration can be, for example, intravenous, topical, subcutaneous, transcutaneous, transdermal, intramuscular, oral, buccal, sublingual, gingival, palatal, intra-joint, parenteral, intra-arteriole, intradermal, intraventricular, intracranial, intraperitoneal, intralesional, intranasal, rectal, vaginal, or by inhalation. By "co-administer" it is meant that an HTG drug is administered at the same time, just prior to, or just after the administration of a second drug (*e*.*g*., another HTG drug, a drug useful for reducing the side-effects of the HTG drug, *etc*.).

A therapeutically effective amount of an HTG drug may be administered repeatedly, *e.g*., at least 2, 3, 4, 5, 6, 7, 8,9, 10 or more times, or the dose may be administered by continuous infusion. The dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" includes physically discrete units suitable as unitary dosages for human subjects and other mammals, each unit containing a predetermined quantity of an HTG drug calculated to produce the desired onset, tolerability, and/or therapeutic effects, in association with a suitable pharmaceutical excipient (*e*.*g*., an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the HTG drug.

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, andpropyl-hydroxy-benzoates (*i.e.,* the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

As described herein, the therapeutically effective dose takes the form of a pill, tablet, or capsule, and thus, the dosage form can contain, along with a HTG drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof A HTG drug can also be formulated into a suppository disposed, for example, in a polyethylene glycol (PEG) carrier.

Liquid dosage forms can be prepared by dissolving or dispersing a HTG drug and optionally one or more pharmaceutically acceptable adjuvants in a carrier such as, for example, aqueous saline (*e.g.,* 0.9% w/v sodium chloride), aqueous dextrose, glycerol, ethanol, and the like, to form a solution or suspension, *e*.*g*., for oral, topical, or intravenous administration. A HTG drug can also be formulated into a retention enema.

Preferably, injectable solutions are formulated at a pH of from about 4.5 to about 7.5.

The therapeutically effective dose can also be provided in a lyophilized form. Such dosage forms may include a buffer, *e*.*g*., bicarbonate, for reconstitution prior to administration, or the buffer may be included in the lyophilized dosage form for reconstitution with, *e*.*g*., water. The lyophilized dosage form may further comprise a suitable vasoconstrictor, *e*.*g*., epinephrine. The lyophilized dosage form can be provided in a syringe, optionally packaged in combination with the buffer for reconstitution, such that the reconstituted dosage form can be immediately administered to an individual.

### D. Dietary interventions

As described herein, dietary interventions are administered to an individual at risk of developing HTG or diagnosed with HTG. Such interventions include a personalized diet, weight management (weight loss) program, increased physical activity, behavioral modification, and caloric restriction. As described herein, the dietary interventions include a restriction of carbohydrates and/or fats in the diet.

As described herein, the dietary intervention is a low-carbohydrate diet wherein carbohydrates are less than 20% of the daily caloric intake. A low-carbohydrate diet can be a diet that restricts carbohydrate intake to about 20 to 60 grams per day.

As described herein, the dietary intervention is a low-fat diet wherein fat is about 20-30% or less of the daily caloric intake.

### E. Nutritional interventions (dietary supplements)

As described herein, nutritional interventions include, for example, phytosterols such as long chain polyunsaturated fatty acids (*e*.*g*., omega-3 polyunsaturated fatty acids such as eicosapentaenoic acid (EPA) and docosahexaenoic acid (DHA), omega-6 polyunsaturated fatty acids, and alpha linolenic acid) and monounsaturated fatty acids, taurine, phytochemicals, phytosterols (*e.g*., plant sterols and plant stanols), probiotics (*e.g*., *Lactobacillus Acidophilus, Lactobacillus plantarum, Lactobacillus reuteri, Bifidobacrium lactis, Bifidobacrium longum),* carbohydrates (*e.g*., resistant starch, beta-cyclodextrin), proteins (*e.g*., soy protein, beta-conglycinin, glycinin), dietary fiber (*e*.*g*., psyllium, pectin, beta-glucan, inulin), phytonutrients/phytochemicals (*e*.*g*., isoflavone, grape polyphenols such as flavonoids, flavonols, anthocyanins, tannins, and resveratrol, punicalagins, catechins), herbs, spices, and combinations thereof. Additional nutritional interventions include, but are not limited to, fructooligosaccharides (FOS), partially-hydrolyzed guar gum (PHG6), a PHG6/FOS blend, fish oil, polyunsaturated fatty acids (PUFA) such as n-6 fatty acids and n-3 fatty acids, choline, niacin, resistant starch, eicosanoids, palmitoleic acid, prebiotics, yeast, antibiotics, food products, drink products, and combinations thereof.

As described herein, the nutritional interventions are tube feed formulations or oral nutritional supplements (*e*.*g*., liquid or solid). As described herein, the nutritional interventions are food products or drink products.

The nutritional intervention can be administered in a formulation, at a dosage and for a duration that is beneficial for the reduction of elevated triglycerides in an individual with hyperlipidemia, *e*.*g*., hypertryglyceridemia.

As described herein, the therapeutically effective amount of the nutritional intervention is administered alone. Also described herein, the nutritional intervention is administered in combination with therapeutically effective amount of a therapeutic drug. Non-limiting examples of a therapeutic drug include a fibrate, such as, for example, bezafibrate (Bezalip), ciprofibrate (Modalim), clofibrate(Abitrate, Atromid-S), gemfibrozil (Lopid), fenofibrate (Lifibr, Antara, Lipofen, Triglide,TriCor), and combinations thereof. Fibrates include any fibric acid derivative agent that can be used to lower plasma lipid levels.

As described herein, the nutritional intervention is a source of phytosterols. Physterols includes plant sterols and stanol esters The formulations of the nutritional intervention can contain limited amounts of saturated fatty acids *(e.g.,* less than 7% of the total energy of the intervention), cholesterol (*e*.*g*., less than 200 mg), and trans fatty acids.

As described herein, the nutritional intervention is a source of long chain polyunsaturated fatty acids (LC-PUFA). The nutritional supplement can be 2 to 4 grams per day of omega-3 fatty acids such as EPA and DHA, or omega-3 fatty acids from marine fish. As described herein, the supplement can be fish oil at a dose of about 3 to 18 grams per day such as 3, 4, 5, 6, 7, 8, 9, 10, 11, 12, 13, 14, 15, 16, 17, or 18 grams per day. The intervention can be a diet rich (high) in omega-3 or omega-6 polyunsaturated fatty acids.

In certain instances, and especially if a subject has elevated triglyceride levels, n-3 fatty acids can be provided. In certain instances, a PHG6/FOS blend is especially preferred.

Prebiotics include food substances intended to promote the growth of probiotic bacteria in the intestines. For instance, a prebiotic can be dietary fibers. Dietary fibers are fibers that are non-digestible by the human body. Non-limiting examples of dietary fibers include fructooligosaccharides, galacto-oligosaccharides, xylo-oligosaccharides, isomalto-saccharides, soya oligosaccharides, pyrodextrins, transgalactosylated oligosaccharides, lactulose, beta-glucan, inulin, raffinose, stachyose, and combinations thereof.

Probiotics refer to microbial cell preparations or components of microbial cells with a beneficial effect on the health or well-being of the host individual. Non-limiting examples of a probiotic bacteria include *Bifidobacterium, Lactobacillus, Lactococcus, Enterococcus, Streptococcus Ascomycota, Deuteromycota, Debaryomyces, Kluyveromyces, Saccharoymces, Yarrowia, Zygosaccharomyces, Candida, Rhodotorula, Bifidobacterium longum* (NCC490; CNCM I-2170; NCC2705; CNCM I-2618), *Bifidobacterium lactis* (NCC2818; CNCM 1-3446; Bb12), *Bifidobacterium animalis, Bifidobacterium breve, Bifidobacterium infantis, Lactobacillus acidophilus, Lactobacillus casei, Lactobacillus salivarius, Lactococcus lactis, Lactobacillus reuteri, Lactobacillus rhamnosus* (NCC4007; CGMCC 1.3724), *Lactobacillus rhamnosus GG* (ATCC53103), *Lactobacillus paracasei* (NCC2461; CNCM I-2116), *Lactobacillus johnsonii* (NCC533; CNCM I-1225), *Lactobacillus plantarum, Lactobacillus salivarius, Enterococcus faecium, Saccharomyces cerevisia, Saccharomyces boulardii, Enterococcus faecium SF 68* (NCIMB10415), and mixtures thereof. It has been shown in a study of type 2 diabetic individuals who were moderately hypercholesterolemic that daily consumption of probiotic yogurt containing *Lactobacillus acidophilus* and *Bifidobacterium lactis* for 6 weeks decreased LDL-cholesterol levels (Ejtahed et al., J Dairy Sci, 94(7):3288-94 (2011)).

Antibiotics include a class of aminoglycoside antibiotics such as, but not limited to, neomycin, kanamycin, streptomycin, a class of polypeptide antibiotics such as polymyxin B, a class of penicillin beta-lactam such as ampicillin, and a class of quinolone/fluoroquinolone antibiotics such as ciprofloxacin and norfloxacin. Also, a bacteriophage or combinations of different bacteriophages may be used. Bacteriophages may be advantageously used when there is antibiotic resistance. Bacteriophages are viruses that invade bacterial cells and, in the case of lytic phages, disrupt bacterial metabolism and cause the bacterium to lyse. Bacteriophage can be used to treat pathogenic bacterial infections. As most phages are specific for one species of bacteria, a single bacteriophage can be used or combinations of different bacteriophages can be used.

As described herein, the nutritional intervention is a source of protein. For instance, any suitable dietary protein can be used, *e*.*g*., animal proteins (such as milk proteins, meat proteins and egg proteins), vegetable proteins (such as soy protein, wheat protein, rice protein, and pea protein), free amino acids (*e*.*g*., taurine), or combinations thereof.

The biological effects of soy protein consumption and the reduction of circulating lipids and the risk of developing cardiovascular disease are fairly established. For instance, it was shown that a soy protein supplementation significantly associated with reducing plasma triglyceride levels compared to carbohydrates (Wofford et al., Eur J Clin Nutr, 66(4):419-25 (2012)) and with lowering LDLcholesterol levels (Anderson et al., J Am Coll Nutr, 30(2):79-91 (2011)). The constitutents of soy protein including soy isoflavone, beta-conglycinin, and glycinin also reduced circulating TGs in animal models (de Souza Ferreira et al., J Func Foods, 2:275-283 (2010)).

Data from animal and human studies suggest a positive effect of taurine (2-aminoethanesulfonic acid) on cholesteroal levels, and potentially plasma triglycerides (Mizushima et al., Adv Exp Med Biol, 403:615-22 (1996); Lombardini and Militante, Adv Exp Med Biol, 583, 251-254 (2006)).

As described herein, the nutritional agent is a source of carbohydrates. Non-limiting examples of a carbohydrate include resistant starch, β-cyclodextrin, sucrose, lactose, glucose, fructose, corn syrup solids, maltodextrins, and mixtures thereof.

Resistant starch such as high-amylose cornstarch is any starch that is not digested in the small intestines, but rather, passes to the large bowel where it is fermented by the gut microbiota. One study showed that high-amylose cornstarch reduced TG concentrations in an animal model of hyperlipidemia (Liu et al., J Nutri Biochem, 21(2):89-97 (2010)). In another study the supplementation of β-cyclodextrin to a high cholesterol diet decreased plasma TG levels in rats (Garcia-Mediavilla et al., Pharmacol Toxicol, 92(2):94-9 (2003); Favier et al., Metabolism, 44(2):200-6 (1995)).

As described herein, the nutritional agent is a source of dietary fiber. Dietary fiber includes, but is not limited to, psyllium, pectin, β-glucan from oat bran or barley, fructooligosaccharides (*e*.*g*., inulin), pectin, guar gum, acacia gum, galacto-oligosaccharides, sialyl-lactose, oligosaccharides derived from milk and any dietary fiber from fruits, vegetables, nuts and grains, and combinations thereof.

Low-fat, high carbohydrate diets commonly increase plasma TG concentrations, however, interventions containing dietary fiber can counteract these effects. For instance, the addition of inulin to a moderately high carbohydrate diet reduced plasma TG concentration in healthy individuals (Letexier et al., Am J Clin Nutr, 77(3):559-64). It has been shown that psyllium comsumption lowered TG in subjects with type 2 diabetes (Sartore et al., Eur J Clin Nutr, 63(10):1269-71 (2009)) and in obese adolescent subjects (Moreno et al., J Physiol Biochem, 59(3):235-42 (2003)). Furthermore, a meta-analysis study demonstrated that psyllium caused a time- and dose-dependent reduction in serum total cholesterol and LDL cholesterol (Wei et al., Eur J Clin Nutr, 63(7):821-7 (2009)). An epidemiological study of 40 to 60 year old subjects showed that consumption of pectin is inversely associated with serum TG concentration (Wu et al., Am J Clin Nutr, 78(6):1085-91 (2003)). In addition, a fructo-oligosaccharide supplementation to a sucrose diet ameliorated plasma TG and free fatty acid concentrations in an insulin-resistant rat model (Agheli et al., JNutr, 128(8):1283-8 (1998)).

Other nutritional agents that can be used for reducing the amount of enterobacteria in the gut can include phytochemicals, for example, phytonutrients. Phytochemicals refer to plant or fruit derived chemical compounds, and phytonutrients refer to phytochemicals derived from edible plants. Non-limiting phytonutrients and phytochemicals include phytonutrients such as isoflavone, polyphenols from grapes such as flavan, anthocyanin, quercetin, myricentin, kaempferol, and resveratrol, phytochemicals from pomegranates such as punicalagin, elagitannins, perlagonidin, pelargonidin, punicalin, anthocyanins, cyanidin, and ellagic acid, phytochemicals from green tea, oolong tea, or coffee such as indole, polyphenols, epigallocatechin, epigallocatechin gallate, flavonoids, tannins, theaflavin, tannic acid, theophylline, theobromine, anthocyanins, and procyanidin, phytochemicals from cashew apple flavor such as (E)-2-hexenal, phytochemicals from rosemary, such as carnosol, carnosic acid or rosmarinic acid, and mixtures thereof.

As described herein, the nutritional intervention comprises a food product including milk-powder based products, instant drinks, ready-to-drink formulations, nutritional powders; milk-based products (*e.g*., yogurt or ice cream), cereal products, beverages, water, teas (*e.g*., green tea or oolong tea), coffee, espresso based drinks, malt drinks, chocolate flavored drinks, culinary products and soups. As described herein, the nutritional agent is a nutritionally complete formula.

### IV. Examples

The following examples are offered to illustrate, but not limit, the claimed invention.

### Example 1:

A growing body of evidence supports a role of lipopolysaccharide (LPS) in the regulation of insulin sensitivity, yet little is known about the relative contribution of intact LPS from intestinal bacteria to host glucose metabolism. This study shows that the abundance of *Escherichia coli* (*E. coli*) is higher in the distal ileum, cecum and colon of *ob*/*ob* mice compared to lean C57BL/6J mice by a factor of 2.0, 3.1 and 2.9 log CFU/g, respectively. To investigate if the over-abundance of *E. coli* is not just the result of leptin deficiency but partially contributes to the obesity and insulin resistance of *ob*/*ob* mice, *E. coli* was isolated from the feces of *ob*/*ob* mice and administered via drinking water to conventional chow-fed to C57BL/6J mice for a period of 9 weeks. Supplementation of *E. coli* resulted in a persistent elevation of fecal and cecal *E. coli* counts, but this had no effect on body weight, body composition and glucose tolerance of mice. However, a trend towards increased plasma (p = 0.07) and liver triglyceride concentrations (p = 0.051) in the *E*. *coli*-treated mice was observed. More importantly, compared with a vehicle treatment, *E. coli* administration significantly blunted plasma triglycerides appearance by 20 % during an oral lipid tolerance test, and this effect coincided with a 31% increase in lipid contents in the ileum. Altogether, the results demonstrate that residential *E. coli* plays an important role in intestinal lipid metabolism and intestinal lipid accumulation independent of obesity. The findings establish a connection between gut microbiota and homeostatic regulation of dietary lipids.

### Introduction

Type 2 diabetes is a pandemic health issue and the International Diabetes Federation estimated that the worldwide prevalence of the disease reaches 371 million at the end of 2012 [1]. Physical inactivity, excessive adiposity and insulin resistance are likely the contributors to the rapid development of type 2 diabetes, and factors including adipokines, oxidative stress, mitochondrial dysfunction and chronic low grade inflammation have been proposed to influence the pathogenesis of type 2 diabetes [2].

Recent data indicates that the gut microbiota may contribute to the development of type 2 diabetes. Several groups have reported that the type 2 diabetes is associated with an altered gut microbiota [3, 4] and the signature of type 2 diabetes associated gut metagenome is enriched in pathways involved in the transport of sugars, branched-chain amino acids, methane metabolism and xenobiotics degradation and sulphate reduction [5]. In supporting the gut microbiota as a cause to type 2 diabetes, one line of evidence based on the reduction of the gut microbiota shows that mice reared in a germfree condition or by applying antibiotics to insulin resistant *ob*/*ob* and C57BL/6J mice showed significantly enhanced oral glucose tolerance [6, 7]. Another line of evidence focusing on the proinflammatory nature of lipopolysaccharide (LPS) located on the cell wall of gram-negative bacteria demonstrates that LPS is partly responsible for high fat diet-induced insulin resistance [8]. When LPS was chronically infused to mice, this resulted in mild obesity, impaired glucose tolerance and hepatic insulin resistance [8]. These effects of LPS were blunted in CD14-deficient mice that have impaired LPS signaling [8], indicating pathophysiological connections between inflammatory responses to bacterial-derived LPS and the development of insulin resistance.

In a normal physiological condition, a small amount of LPS can be found in circulation, but the concentration of LPS is reportedly higher in type 2 diabetic than in healthy subjects [9, 10]. However, the underlining mechanism responsible for the LPS uptake by the intestinal epithelial cells is not clear. Both transcellular andparacellular [11] pathways have been suggested. Importantly, dietary fat is a critical nutritional factor which facilitates LPS absorption [12]. In mice, Cani *et al.* showed that high fat feeding elevates circulating LPS levels [8]. In healthy individuals, circulating LPS concentrations positively correlate with energy intake [13], and in obese individuals, the degree of hypertriglyceridemia is positively associated with the LPS concentrations in the serum [14]. Recently, Amar *et al.* showed that high fat feeding results in the adherence of GFP-labelled *E. coli* to the mucosa of the ileum [15], hence suggesting dynamic adaptation of intestinal Gram negative bacteria such as *E. coli* to dietary fat.

*E. coli* is the major facultative anaerobic bacterium in the human intestine. Residential strains of *E. coli* which are usually harmless in a nutrient-rich and carbon-rich intestinal environment can become pathogenic when going into the nitrogen-rich urinary track [16]. Other strains such as enterotoxic *E. coli* are pathogenic and can cause diarrhea [17]. Contrary to pathogenic *E. coli,* the *E. coli* Nissle 1917 strain can act probiotic, for instance by ameliorating gastrointestinal disorder [18]. Recently, Santacruz *et al.* showed that a higher level of *E. coli* was found in gut microbiota of overweight pregnant women than normal-weight counterparts [19].

To investigate the role of residential *E*. *coli* in insulin resistance independent of high fat feeding, a series of experiments were conducted, first by examining the abundance of *E. coli* in different part of intestine of the obese and insulin resistant *ob*/*ob* mice fed a chow diet. Then, the *E. coli* was isolated from the feces of *ob*/*ob* mice were isolated and administered to conventional chow-fed C57BL/6J mice. Glucose and lipid metabolism of the treated mice were examined and data are presented herein.

### Materials and Methods

### Examination of luminal content in ob/ob mice versus lean mice.

Six weeks old C57BL/6J and *ob*/*ob* mice were purchased from Charles River Laboratories (L'Arbresle, France). The mice were housed in groups (5 mice per cage) and had free access to chow diet (Kliba 3437, Provimi Kliba Nafag, Switzerland) and water under a 12h/12h light/dark cycle After 3 weeks of habituation, mice were killed and their gastrointestinal tract was collected and cut into 9 segments of about 2.5 cm length from stomach to rectum. Segments were weighed and immediately frozen in liquid nitrogen for quantitative PCR.

### Colonization of mice by E. coli derived from ob/ob mice.

Colonization of conventional lean mice by *ob*/*ob* mice-derived *E*. *coli* was investigated in a set of 3 protocols. For each, 8-week old male C57BL/6J mice were purchased from Charles River Laboratories (L'Arbresle, France). The mice were housed individually and had free access to chow diet (Kliba 3437, Provimi Kliba Nafag, Switzerland) and Vittel water under a 12h/12h light/dark cycle. After 2 weeks habituation, the mice were assigned into one of the two weight-matched groups. Group 1 received Vittel water and group 2 received the addition of 10⁸ CFU/mL *E. coli* in drinking water for 65 days. Body weight, water and food intake were recorded twice a week. Body composition was measured in a conscious state using EchoMRI TM 3-in-1 (Echo Medical Systems, Houston, TX) at baseline (day 0) and towards the end of the supplementation period (day 63). *Enterobacteriaceae* were enumerated from feces at days -3, +3, and then every two weeks during the treatment period. At the end of the treatment, mice were either: 1) killed after overnight fasting, 2) subjected to an oral glucose tolerance test (OGTT) after 6 hours of fasting, or 3) challenged with an oral lipid tolerance test (OLTT) after overnight fasting. All mice were killed at day 66. Blood glucose was measured by tail incision before the mice were anesthetized by isoflurane. Blood samples were collected in EDTA coated tubes from vena cava and plasma was collected by centrifugation and stored at -40°C until analysis. Adipose tissues, liver, intestine and cecum content were weighted, flash frozen in liquid nitrogen and stored in a minus 80 °C freezer for further analysis. Two independent experiments were performed and a total number of 12 animals were used per experimental group.

### Isolation, preparation and detection of E. coli from the feces of ob/ob mice

Feces from 8 *ob*/*ob* mice were plated *on Enterobacteriaceae* specific Drigalski medium. One to two *Enterobacteriaceae* colonies from each mouse were selected and analyzed with the API20E gallery (Biomerieux, France). DNA from the same colonies were compared by enterobacterial repetitive intergenic consensus-PCR (ERIC-PCR; *see, e.g.,* Dalla-Costa et al., J. Med. Microbiol., 47:227-234 (1998)) and repetitive extragenic palindromic (REP) PCR for genetic identity [20, 21].

A selected *E. coli* colony was enumerated in aerobic suspensions prepared with LB medium. Twice a week, a fresh *E. coli* suspension was prepared from the frozen glycerol stock by adding the pre-culture to 15 mL LB medium. The pre-culture was allowed to grow for 6-8 hours at 37 °C. Thereafter, 2.5 mL of the saturated pre-culture solution was added to two flasks filled with 250 mL of LB medium and the cultures were allowed to grow overnight. The following morning, the bacterial solutions were centrifuged for 5 min at 5000xg (4 °C) and the bacterial pellet was re-suspended in a final volume of 2.5 L Vittel water, which was then given to the mice. To confirm successful colonization with *E. coli, E. coli* levels in feces were quantified by plating different dilutions of a fresh feces suspension on a semi-selective culture medium (Drigalski growth medium) for enrichment. In addition, cecal *E. coli* was quantified using quantitative PCR with *E*. coli-specific primers and compared with total bacteria content. Primers and conditions for PCR amplification are represented in Table 1.

**Table 1. Sequences of the primers and PCR conditions used for detection of bacteria**

| **Strain** | **SEQ ID NO:** | **Primers sequence (5'-3')** | **PCR conditions** |
|---|---|---|---|
| Total bacteria | SEQ ID NO:1 | F: TCCTACGGGAGGCAGCAGT | 50°C, 2 min (1x) / 95°C, 10 min (1x) / 95°C, 15 sec + 60°C, 1 min (40x) |
| | SEQ ID NO:2 | R: GGACTACCAGGGTATCTAATCCTGTT | |
| *Escherichia coli* | SEQ ID NO:3 | F:CATGCCGCGTGTATGAAGAA | 50°C, 2 min (1x) / 95°C, 10 min (1X) / 95°C, 15 sec + 60°C, 1 min (40x) |
| | SEQ ID NO:4 | R: CGGGTAACGTCAATGAGCAAA | |
| *Firmicutes* | SEQ ID NO:5 | F: GGAG(CT)ATGTGGTTTAATTCGAAGCA | 50°C, 2 min (1x) / 95°C, 10 min (1x) / 95°C, 15 sec + 60°C, 1 min (40x) |
| | SEQ ID NO:6 | R: AGCTGACGACAACCATGCAC | |
| *Lactobacillus* | SEQ ID NO:7 | F: AGCAGTAGGGAATCTTCCA | 95°C, 5 min (1x) / 95°C, 15 sec + 58°C, 20 sec + 72°C, 45 sec (35x) / 72°C, 5 sec (1x) |
| | SEQ ID NO:8 | R:CACCGCTACACATGGAG | |
| *Bacteroidetes* | SEQ ID NO:9 | F: GGA(AG)CATGTGGTTTAATTCGATGAT | 50°C, 2 min (1x) / 95°C, 10 min (1x) / 95°C, 15 sec + 60°C, 1 min (40x) |
| | SEQ ID NO:10 | R: AGCTGACGACAACCATGCAG | |
| *Bacteroidaceae-Prevotella-Porphyromonas* | SEQ ID NO:11 | F: GGTGTCGGCTTAAGTGCCAT | 95°C, 5 min (1x) / 95°C, 15 sec + 68°C, 20 sec + 72°C, 30 sec (35x) / 80°C, 30 sec (1x) |
| | SEQ ID NO:12 | R: CGGA(CT)GTAAGGGCCGTGC | |
| *Bifidobacterium* | SEQ ID NO:13 | F: CTCCTGGAAACGGGTGG | 94°C, 5 min (1x) / 94°C, 20 sec + 55°C, 20 sec + 72°C, 50 sec (40x) / 94°C, 15 sec (1x) |
| | SEQ ID NO:14 | R:GGTGTTCTTCCCGATATCTACA | |
| *Enterobacteriaceae* | SEQ ID NO:15 | F: TGCCGTAACTTCGGGAGAAGGCA | 95°C, 15 min (1x) / 94°C, 20 sec + 60°C, 20 sec + 72°C, 50 sec (40x) / 95°C, 15 sec (1x) |
| | SEQ ID NO:16 | R:TCAAGGCTCAATGTTCAGTGTC | |

| | | | |
|---|---|---|---|
| "F" denotes forward primer. "R" denotes reverse primer. | | | |

### Oral Glucose Tolerance Test (OGTT)

An OGTT was performed at the end of the treatment period after 6 hours food deprivation in the light cycle. After measuring fasting glucose concentration in blood obtained by tail incision using an Ascensia Elite XL glucometer (Bayer AG, Zurich, Switzerland), animals were given a 30% glucose solution at the dose of 2 g/kg (wt/BW) by oral gavage at time 0. Blood glucose was measured after 15, 30, 60 and 120 min. Blood was also collected in EDTA coated tubes at 0, 15 and 60 min for insulin analyses (IBL; Hamburg, Germany).

### Oral Lipid Tolerance Test (OLTT)

In an independent experiment, *E*. *coli*-treated mice and control mice underwent an OLTT after overnight fasting (14 hours). As for the OGTT, the OLTT was performed at the end of the treatment period. Animals received 6 ml/g (wt/BW) of corn oil by oral gavage at time 0. Blood was collected into EDTA-coated tubes at 0, 60, 120, 180 and 240 min for triglycerides (TG) and non-esterified fatty acids (NEFA) analysis. TGs and NEFA were measured using commercial kits from BioMérieux (France) and Wako Diagnostics (Richmond, VA), respectively.

### Plasma and Tissue Analysis

Plasma triglyceride, total cholesterol, non-esterified fatty acids (Wako Diagnostics, Richmond, VA), β-hydroxybutyrate (Wako Chemicals GmbH, Neuss, Germany) were measured using the COBAS C111 (Roche Diagnostics, Mannheim, Germany). Plasma insulin (IBL; Hamburg, Germany) and LPS-binding protein (ELISA-LBP; #CKM043; Cell Sciences, Inc.; Canton, MA) levels were measured using commercial kits. Blood glucose was measured by tail incision using Ascensia Elite XL glucometer (Bayer AG, Zurich, Switzerland). Plasma inflammatory markers were measured on a MSD Mouse TH1/TH2 9-Plex Ultra-Sensitive Kit plate (MesoScale Discovery, Gaithersburg, Maryland). Detection was applied using the manufacturer's instructions.

Lipids were extracted from 200 mg of frozen liver according to Folch *et al.* [22]. Cholesterol was measured using commercial kit (Roche Diagnostics, Mannheim, Germany), following the manufacturer's instructions. TGs were first hydrolyzed in a basic solution (0.5 N KOH in ethanol) and then measured using a commercial enzymatic TG analysis kit (BioMérieux, France), following the manufacturer's instructions.

### mRNA expressions analysis

Total RNAs were prepared from 50 mg liver or intestinal sections using the Agencourt RNAdvance tissue kit (Agencourt, Beckman Coulter, Germany) according to the protocol provided by the manufacturer. Reverse transcription was performed on 1.5 µg total RNA using the 1^{st} strand cDNA synthesis kit for real time PCR (AMV; Roche Biomedical, Basel, Switzerland) with oligo d(T)₁₅ as primer. Real-time reverse transcription-PCR analyses were performed in a fluorescent temperature cycler (GeneAmp^{®} PCR 5700 Sequence Detection System; Applied Biosystems). Primers sequences are listed in Table 2.

**Table 2. Sequences of the primers and PCR conditions for RNA expression analysis.**

| **Gene of interest** | **SEQ ID NO** | **Primers sequence (5'-3')** |
|---|---|---|
| NFκB | SEQ ID NO:17 | F: CGGACAGCCCTCCACCTT |
| | SEQ ID NO:18 | R: CAGCCCCACATTTCAACAAGA |
| Il-6 | SEQ ID NO:19 | F: GAAATGATGGATGCTACCAAACTG |
| | SEQ ID NO:20 | R: CCAGAAGACCAGAGGAAATTTTCA |
| TNFα | SEQ ID NO:21 | F: ACCGTCAGCCGATTTGCTAT |
| | SEQ ID NO:22 | R: TTGACGGCAGAGAGGAGGTT |
| ACC2 | SEQ ID NO:23 | F: AGCGGGACTCTGTCCTCAAG |
| | SEQ ID NO:24 | R: CCATGATGACCTCTGGATGTTCT |
| PPARα | SEQ ID NO:25 | F: ACCATCCAGATGACACCTTCCT |
| | SEQ ID NO:26 | R: GCCGAAGGTCCACCATTTTT |
| MTTP | SEQ ID NO:27 | F: CACCTCAGCCAGAAAGCGATA |
| | SEQ ID NO:28 | R: AGGGAACCCACGGGAATC |

| | | |
|---|---|---|
| "F" denotes forward primer. "R" denotes reverse primer. | | |

### Statistical Analysis

Data are presented as median ± SEMedian. Outcomes taken at a unique time point were assessed by Wilcoxon tests, whereas two-tailed non-parametric tests (Mann-Whitney tests) were used to compare treatment versus control groups. In order to analyze the effect *of E. coli* on body weight and body weight gain, mixed effects modeling was applied (repeated measurements).

### Results

*E. coli* levels are increased in the distal part and in feces of genetically obese *(ob*/*ob)* mice. Luminal contents of *ob*/*ob* and wild type mice on a C57BL/6J background were examined by a quantitative PCR technique, and the results showed that total bacteria number was similar in *ob*/*ob* and wild type mice (Table 3) while the proportion of *E. coli* in the distal part of the gut (*i.e.,* cecum, colon and feces) was higher in *ob*/*ob* than in their lean counterparts by 2.0, 3.1 and 2.9 log CFU/g, respectively. To assess whether the over-represented strain could be functionally linked to obesity, *E. coli* was isolated from these mice. To this end, feces from 8 different *ob*/*ob* mice was added to *Enterobacteriaceae*-selective culture medium and grown under selective conditions. All growing colonies were identified as *E. coli* by analysis on the API20E gallery. Genetic identity of all the clones were compared with ERIC- and REP-PCR, and results showed that all colonies had identical molecular signatures. Since these techniques did not show any difference between the different isolates, a single colony from the mouse displaying the highest body weight was picked for the following experiments.

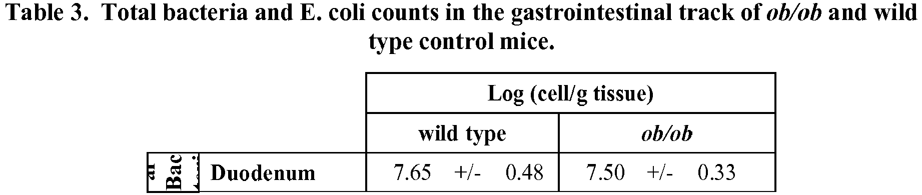

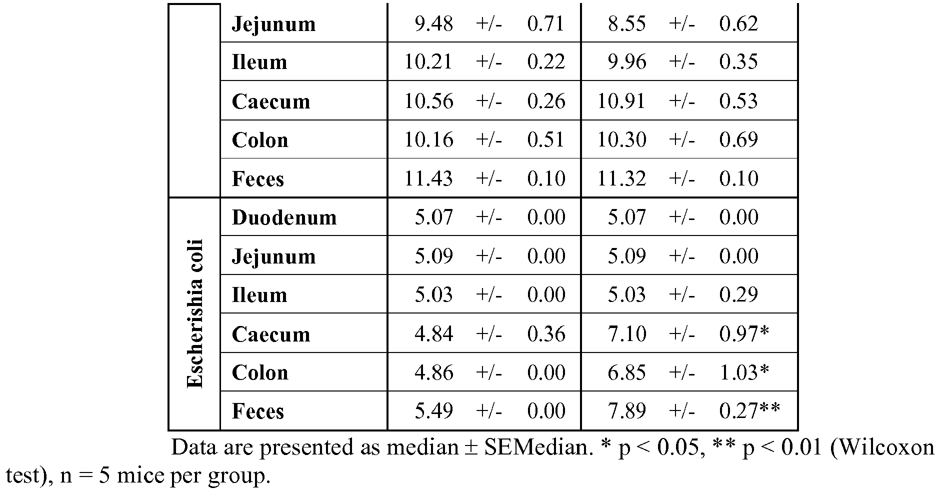

To test the possible physiological impacts of *E*. *coli* strain isolated from ob/ob mice, wild type conventional chow-fed mice were given access to water containing 10⁸ CFU/mL of *E. coli* for 65 days. Water intake was measured twice a week, and no difference was found with supplementation of *E. coli* (Table 4).

**Table 4. Cumulative water and food intake/efficiency.**

| | **Control** | **Treated** |
|---|---|---|
| **Cumulative water intake (g)** | 88.1 ± 3.2 | 77.9 ± 3.9 |
| **Cumulative food intake (Kcal)** | 1380 ± 121 | 1536 ± 279 |
| **Food efficiency (mg/Kcal)** | 4.04 ± 0.42 | 3.95 ± 0.43 |

| | | |
|---|---|---|
| Data are presented as median ± SEMedian, n=11-12. | | |

Based on the water consumption, the average intake of *E. coli* was around 8.6 ± 0.2 Log CFU/day. Success of colonization was monitored by culture-based enumeration *of E. coli* from the feces *of E. coli*-treated mice at different time-points during the study. As shown in Table 5, the treated mice exhibited an increase in fecal *E. coli* counts and the elevated *E. coli* counts persisted during the course of experiment (up to day 62).

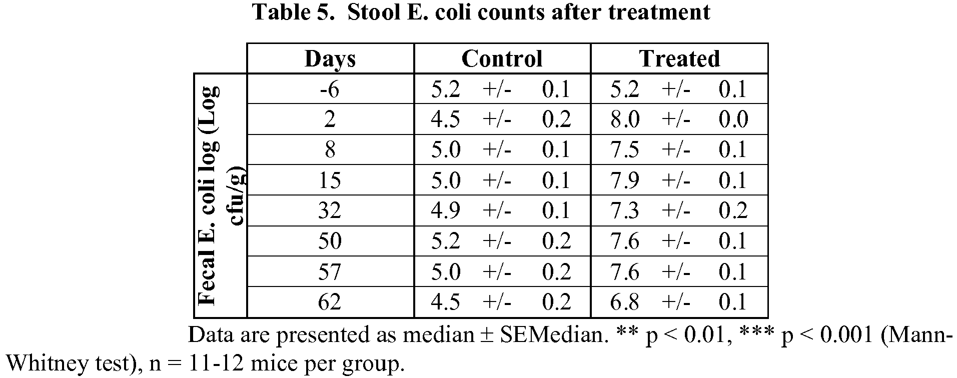

The increase of *E. coli* colonization was specific. The amount of total fecal bacteria, *Firmicutes, Bacteroidetes, Enterobacteriaceae, Bacteroidetes-Prevotella-Porphyromona, Bifidobacteria,* and *Lactobacillus* groups was determined by a quantitative PCR technique, and all groups except for *Enterobacteriaceae* (the family where *E. coli* belongs to) were unaffected by the *E. coli* supplementation (Table 6).

**Table 6. Amount of bacterial groups at the beginning and at the end of the study.**

| | **Control** | | **Treated** | | **Detection limit** |
|---|---|---|---|---|---|
| **Log (cell/g)** | **Initial** | **Final** | **Initial** | **Final** | |
| **Total bacteria** | 10.8 ± 0.1 | 10.6 ± 0.1 | 10.7 ± 0.1 | 10.6 ± 0.1 | 6.021 |
| ***Firm icutes*** | 10.5 ± 0.1 | 10.6 ± 0.1 | 10.6 ± 0.1 | 10.6 ± 0.1 | 6.224 |
| ***Bacteroidetes*** | 10.6 ± 0.1 | 10.6 ± 0.1 | 10.6 ± 0.1 | 10.6 ± 0.1 | 5.890 |
| ***Enterobacteriaceae*** | 7.0 ± 0.0 | 7.0 ± 0.0 | 7.0 ± 0.0 | 9.1 ± 0.1 | 6.993 |
| ***Bacteroidetes*-*Prevotella-Porphyromona*** | 9.7 ± 0.1 | 9.9 ± 0.2 | 9.9 ± 0.1 | 9.9 ± 0.1 | 7.958 |
| ***Bifidobacteria*** | 8.6 ± 0.2 | 7.0 ± 0.0 | 9.6 ± 0.4 | 8.6 ± 0.2 | 8.306 |
| ***Lactobacillus*** | 9.5 ± 0.1 | 10.6 ± 0.1 | 10.6 ± 0.1 | 10.6 ± 0.1 | 6.175 |

| | | | | | |
|---|---|---|---|---|---|
| Units are Log cell/g. Data are presented as median ± SEMedian, n=11-12. | | | | | |

It has been reported that plasma LPS binding protein (LBP) levels correlate with the amount of plasma LPS [23]. Therefore, LBP was used as a surrogate marker for the systemic exposure to LPS. LBP was found in both groups but the concentrations were elevated in *E. coli-*treated as compared with the control animals (Table 7). Thus, *E. coli* administration in drinking water led to increased *E*. *coli* population in the recipient mice, demonstrating successful and persistent colonization *of E. coli* in these mice.

**Table 7. Cecal E.coli count and plasma lipopolysaccharide binding protein concentration in the treated and untreated mice**

| | **Control** | **Treated** |
|---|---|---|
| **Cecal E. coli (Log CFU/g)** | 6.41 +/- 0.018 | 8.15 +/- 0.01*** |
| **Plasma LBP (µg/ml)** | 5.04 +/- 0.15 | 7.95 +/- 0.39** |

| | | |
|---|---|---|
| Data are presented as median ± SEMedian. ** p < 0.01, *** p < 0.001 (Mann-Whitney test), n = 11-12 mice per group. | | |

Plasma proinflammatory cytokines were measured in mice and none of the parameters (IFN-y, IL-1β, IL-2, IL-4, IL-5, KC-GRO, IL-10, IL-12-p70 and TNFα) was found different between the *E. coli* treated and control mice in an overnight fasting state (Table 8). Jejunal NF-κB, IL-6 and TNFα mRNA expressions were also measured and no difference was found between the two groups (Table 8 and Table 9).

**Table 8. Plasma cytokines after overnight fast.**

| **pg/ml** | **Control** | **Treated** |
|---|---|---|
| **IFN-γ** | 1.2 ± 0.1 | 0.8 ± 0.2 |
| **IL-1β** | 9.2 ± 0.4 | 6.1 ± 0.7 |
| **IL-2** | 9.6 ± 0.7 | 8.0 ± 1.4 |
| **IL-4** | 3.6 ± 0.9 | 2.5 ± 0.8 |
| **IL-5** | 6.8 ± 0.6 | 8.5 ± 0.4 |
| **KC-GRO** | 54.2 ± 4.7 | 55.3 ± 7.7 |
| **IL-10** | 35.7 ± 3.4 | 23.2 ± 2.2 |
| **IL12-p70** | 1083.4 ± 75.6 | 1211.5 ± 142.5 |
| **TNFα** | 490.8 ± 36.0 | 295.5 ± 30.5 |

| | | |
|---|---|---|
| Units are pg/ml. Data are presented as median ± SEMedian, n=11-12. | | |

**Table 9. Jejunal TNFa expression after overnight fast.**

| **Relative to RPLP0** | **Control** | **Treated** |
|---|---|---|
| **NF-κB** | 1.6 ± 0.2 | 1.8 ± 0.4 |
| **IL-6** | 4.4 ± 0.2 | 5.7 ± 0.2 |
| **TNFα** | 1.2 ± 0.2 | 1.2 ± 0.4 |

| | | |
|---|---|---|
| Data are presented as median ± SEMedian, n=12. | | |

At the start of the experiment, both groups displayed the same body weight (25.2 ± 0.3 g in *E*. *coli*-treated mice versus 25.3 ± 0.3 g in control mice), and weight gain in the course of the study was not affected by the *E. coli* treatment ( Table 10).

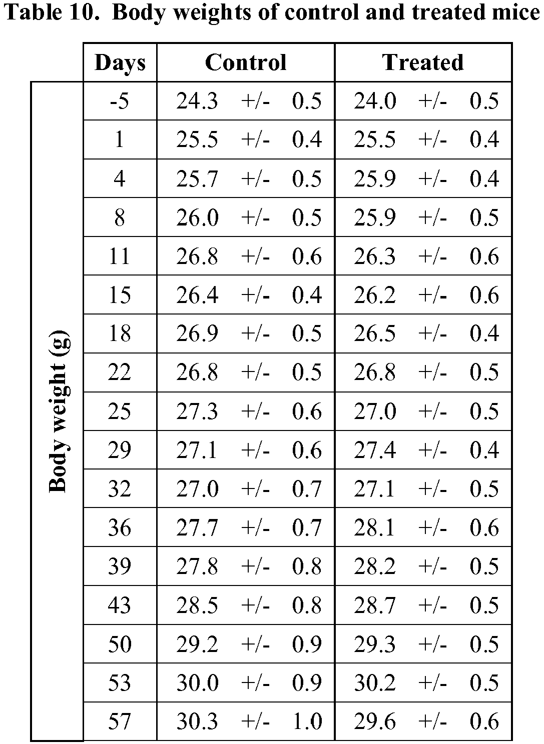

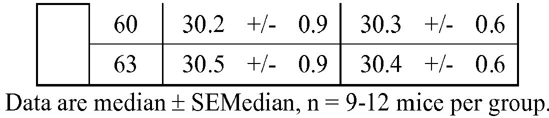

Body composition was measured before and after the treatment and the results showed no difference in lean and fat mass between *E*. *coli*-treated animals and the respective controls (Table 11). *E. coli*-treated mice displayed normal body weight and glucose tolerance.

**Table 11. Body composition of the treated and untreated mice.**

| | **Control** | | **Treated** | |
|---|---|---|---|---|
| **Days** | **Lean mass (%)** | **Fat mass (%)** | **Lean mass (%)** | **Fat mass (%)** |
| **-5** | 87.0 +/- 0.8 | 10.0 +/- 0.3 | 87.1 +/- 0.9 | 11.0 +/- 0.7 |
| **63** | 77.7 +/- 1.6 | 15.4 +/- 1.5 | 75.1 +/- 0.7 | 18.1 +/- 1.1 |

| | | | | |
|---|---|---|---|---|
| Data are median ± SEMedian, n = 9-12 mice per group. | | | | |

At the end of the study, mice were challenged with an oral glucose load. As shown in Table 12, fasting glycemia was nearly identical in both groups and glucose tolerance curves showed no difference between treated mice and the control mice. In agreement with the glucose data, plasma insulin values during the oral glucose tolerance test (OGTT) showed no difference between treated and untreated animals (Table 12), indicating that glucose handling in the chow-fed C57BL/6J mice was not affected by the elevated numbers of intestinal *E. coli.*

**Table 12. Blood glucose, plasma insulin and area under the curve of both parameters during an oral glucose tolerance.**

| | **Glucose (mmol/l)** | | **Insulin (ng/ml)** | |
|---|---|---|---|---|
| **Time (min)** | **Control** | **Treated** | **Control** | **Treated** |
| **0** | 7.0 +/- 0.3 | 7.6 +/- 0.2 | 0.9 +/- 0.1 | 1.0 +/- 0.1 |
| **15** | 16.1 +/- 0.9 | 16.7 +/- 0.7 | 2.1 +/- 0.3 | 2.2 +/- 0.3 |
| **30** | 16.4 +/- 1.8 | 16.1 +/- 1.2 | | |
| **60** | 12.3 +/- 1.0 | 12.1 +/- 1.1 | 0.8 +/- 0.1 | 0.7 +/- 0.2 |
| **120** | 9.1 +/- 0.6 | 9.1 +/- 0.4 | | |
| **AUC** | 1481 +/- 128 | 1482 +/- 87 | 80.2 +/- 14.3 | 88.6 +/- 16.0 |

| | | | | |
|---|---|---|---|---|
| Data are presented as median ± SEMedian, n = 9-12 mice per group. AUC denotes area under the curve. | | | | |

Altered lipid profile in plasma and liver *of E.* coli-treated mice. Organ weights of treated and untreated mice at sacrifice were compared after 6 hours food deprivation, and no difference was found between the two groups ( Table 13).

**Table 13. Body and tissues weight at sacrifice**

| | **Control** | **Treated** |
|---|---|---|
| **Body weight (g)** | 27.2 ± 0.5 | 27.2 ± 0.4 |
| **Epididymal A.T. (g)** | 0.647 ± 0.078 | 0.681 ± 0.043 |
| **Retroperitoneal A.T. (g)** | 0.166 ± 0.025 | 0.164 ± 0.012 |
| **Mesenteric A.T. (g)** | 0.216 ± 0.022 | 0.232 ± 0.018 |
| **Total fat pad (g)** | 1.028 ± 0.121 | 1.077 ± 0.072 |
| **Brown A.T. (g)** | 0.076 ± 0.007 | 0.067 ± 0.003 |
| **Gastrocnemius (g)** | 0.332 ± 0.011 | 0.322 ± 0.006 |
| **Liver (g)** | 1.085 ± 0.023 | 1.046 ± 0.018 |
| **Cecum (g)** | 0.272 ± 0.015 | 0.248 ± 0.011 |

| | | |
|---|---|---|
| Data are presented as median ± SEMedian, n=12. | | |

However, a trend was observed towards increased plasma triglycerides (TGs) in the *E*. *coli*-treated group (p = 0.07, Table 14) whereas plasma non-esterified fatty acids (NEFAs) were not affected by the treatment. In addition to the changes in TGs, circulating ketone body β-hydroxybutyrate (β-HB) tended to be higher in the treated than in the untreated group (p= 0.07). In the intestine, expression of jejunal microsomal triglyceride transfer protein (MTTP, a gene responsible for triglyceride containing lipoprotein secretion) followed the plasma TG levels and showed a trend to be increased (+37%) in the treated mice compared with control mice (10.8e-2 ± 1.8e-2 vs. 8.6e-2 ± 0.9e-2, respectively, relative to GAPDH, p = 0.069). In the liver, TG concentrations were similarly enhanced in the *E*. *coli*-treated mice by a factor of 24 % (p = 0.051, Table 14). Since the amount of β-HB reflects the degree of fatty acid oxidation, the mRNA expression of several lipid oxidation genes in the liver was examined. Acetyl-CoA carboxylase 2 (ACC2), an enzyme regulating in the synthesis cytosolic malonyl-CoA [24], and the concentration of cytosolic malonyl CoA negatively regulates the lipid oxidation by inhibiting the mitochondrial long chain fatty acid transporter CPTla. The mRNA expression of ACC2 was decreased in the treatment group (Table 14), which suggests the increase of fatty acid uptake by mitochondria for oxidation. In addition, peroxisome proliferator-activated receptor α (PPARα), the gene responsible for turning on the lipid oxidation program [25], showed a trend for increased expression in the *E. coli* treated mice (Table 14).

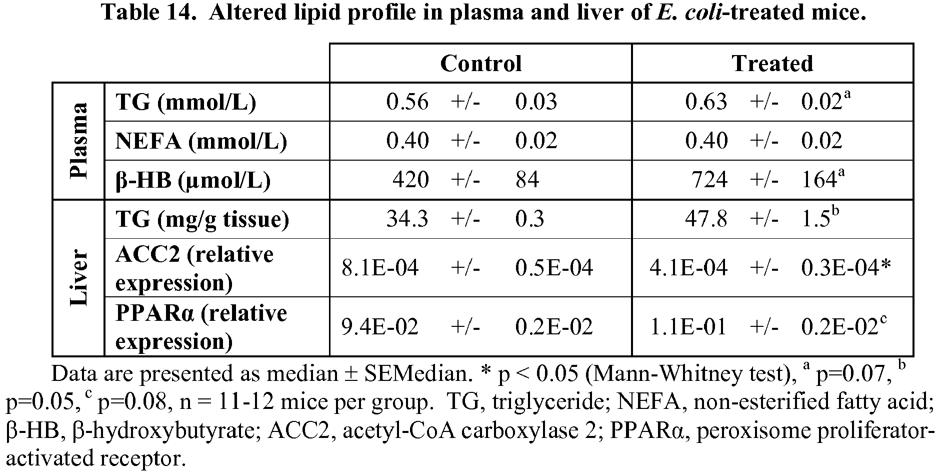

Thus, *E. coli* treatment selectively affected the hepatic lipid metabolism by promoting hepatic fatty acid β-oxidation in a 6-hour fasting state without changing the body composition and oral glucose tolerance of mice, but it seemed to. However, such effect *of E. coli* was sensitive to prolonged fasting as plasma TG, β-HB and NEFA levels as well as hepatic lipid contents were comparable between the *E. coli* treated and untreated mice after 14 hours overnight fasting (Table 15).

**Table 15. Lipids measured after 14 hours overnight fasting.**

| | **Control** | **Treated** |
|---|---|---|
| **Plasma** | | |
| **Triglycerides** (**mmol/L**) | 1.02 ± 0.08 | 1.05 ± 0.07 |
| **NEFA (mmol/L)** | 0.60 ± 0.02 | 0.60 ± 0.05 |
| **β-Hydroxybutyrate (µmol/L)** | 2200 ± 133 | 2200 ± 167 |

| **Liver** | | |
|---|---|---|
| **Triglycerides (mg/g dry)** | 438.8 ± 11.6 | 429.6 ± 15.1 |

| | | |
|---|---|---|
| Data are presented as median ± SEMedian, n=12. | | |

To further investigate the *E*. *coli*-induced changes in lipid metabolism, the mice were challenged with an oral lipid tolerance test (OLTT). To this end, 14-hour fasted mice were orally gavaged with a lipid load and plasma TG concentrations were measured over a period of 4 hours. As expected, plasma TG levels were increased during the OLTT in control mice, peaking at 60 min post gavage (Table 16). In contrast, *E*. *coli*-treated mice did not show the expected increase in plasma TGs, and TG concentrations remained near the baseline throughout the 4 hours observation. NEFA concentrations, on the other hand, were reduced after the oral lipid gavage and the degree of suppression was nearly identical in treated and control mice (Table 16).

**Table 16. Triglyceride and non-esterified fatty acid concentrations during an oral lipid challenge.**

| | **TG (mmol/l)** | | **NEFA (mmol/l)** | |
|---|---|---|---|---|
| **Time (min)** | **Control** | **Treated** | **Control** | **Treated** |
| **0** | 1.05 +/- 0.06 | 1.02 +/- 0.04 | 1.56 +/- 0.08 | 1.66 +/- 0.08 |
| **60** | 1.22 +/- 0.08 | 1.02 +/- 0.07 | 1.20 +/- 0.06 | 1.09 +/- 0.12 |
| **120** | 1.23 +/- 0.08 | 0.94 +/- 0.04* | 1.31 +/- 0.07 | 1.13 +/- 0.11 |
| **180** | 1.18 +/- 0.12 | 0.90 +/- 0.05** | 1.27 +/- 0.07 | 1.21 +/- 0.09 |
| **240** | 1.09 +/- 0.06 | 0.94 +/- 0.06 | 1.32 +/- 0.04 | 1.38 +/- 0.10 |
| **AUC** | 287 +/- 11 | 230 +/- 11** | | |

| | | | | |
|---|---|---|---|---|
| Data are presented as median ± SEMedian. *p<0.05 compared with control group, **p<0.01 compared with control group. (Mann-Whitney test), n=11-12 mice per group. | | | | |

Post OLTT plasma parameters indicated that this effect was specific to TGs, as other lipid classes such as NEFA or cholesterol were not affected by the treatment (Table 17). After the OLTT, a significant amount of lipid was found in the cecum but the amounts were similar in the two groups of animals (Table 18). However, ileal TGs were found to be significantly elevated in E. *coli*-treated mice (Table 18), suggesting that retention of TGs by the intestine is the cause for the apparent non-responsiveness of treated mice to an oral lipid load. *E. coli-*treated mice are characterized by abnormal response to oral lipid load and lipid accumulation in ileum.

**Table 17. Plasma lipids measured after OLTT.**

| | **Control** | **Treated** |
|---|---|---|
| **Triglycerides** (**mmol**/**L**) | 1.59 ± 0.10 | 1.18 ± 0.08* |
| **NEFA (mmol/L)** | 0.60 ± 0.05 | 0.65 ± 0.05 |
| **Cholesterol (mmol/L)** | 2.28 ± 0.10 | 2.35 ± 0.02 |
| **β-Hydroxybutyrate (µmol/L)** | 3125 ± 138 | 3145 ± 188 |

**Table 18. Concentrations of triglyceride in the cecal content and in the ileum.**

| | **Control** | **Treated** |
|---|---|---|
| **Cecum TG (mg/g dry)** | 372.2 +/- 8.8 | 319.5 +/- 16.0 |
| **Ileum TG (mg/g wet)** | 21.6 +/- 2.9 | 28.2 +/- 2.3* |

Data are presented as median ± SEMedian. * p<0.05 compared with control group, ** p<0.01 compared with control group. (Mann-Whitney test), n=11-12 mice per group.

To determine if the correlation between increased plasma triglycerides and an abundance of intestinal *E. coli,* as observed in the mouse study (Table 3 and Table 14), was also true in human subjects with Type 2 diabetes, plasma TG concentration and stool *E. coli* counts were measured in overweight, diabetic and overweight, non-diabetic patients. The diabetic subjects showed a statistically significant increase in plasma TG levels as well as in *E.coli* counts (Table 19). The data indicates that detecting a higher level (*e*.*g*., concentration) of intestinal *E*. *coli* can be predictive or increased plasma TG and/or diabetes in an overweight subject.

**Table 19. Plasma triglyceride concentration and stool E. coli counts of overweight diabetic and overweight non-diabetic human subjects**

| | Overweight Diabetic | Overweight non-diabetic |
|---|---|---|
| Plasma triglyceride (mmol/L) | 1.54 +/- 0.21*** | 0.99 +/- 0.10 |
| Stool E. coli (cell/g) | 7.48 +/- 0.27*** | 7.06 +/- 0.22 |

| | | |
|---|---|---|
| Data are presented as median ± SEMedian. *** p < 0.001 compared with overweight non-diabetic group. (Mann-Whitney test), n = 21-25 per group. | | |

### Discussion

The proinflammatory nature of LPS has been well-documented for causing insulin resistance. However, less is known if increasing luminal quantity of LPS by providing viable *E*. *coli* can impair the insulin sensitivity. Therefore, *E. coli* was isolated from *ob*/*ob* mice and supplemented to conventional C57BL/6J mice. In contrast to recent publications with LPS, no significant change in body weight and oral glucose tolerance was observed in mice following treatment. Instead, intestinal lipid metabolism in response to an oral lipid challenge was dramatically altered.

Lipopolysaccharides (LPS) in the outer membrane of gram-negative bacteria have been shown to participate to the etiology of metabolic disorders [8]. In this study, higher levels of gram-negative *E. coli* was observed in the distal ileum, cecum and colon of *ob*/*ob* than in that of wild type mice. Presumably, the LPS on the surface of residential *E. coli* can enter the general circulation due to the impaired GLP-2-mediated intestinal barrier function in *ob*/*ob* mice [26] as it has been shown that *ob*/*ob* mice have higher concentrations of circulating LPS than wild type control [27]. Instead of measuring LPS. LBP has been proposed as a surrogate marker for LPS as the concentrations of plasma LBP and LPS are both higher in the *ob*/*ob* than control mice [23]. In the present study, LBP levels were higher in the *E. coli* treated mice at a post-absorptive state as well as after the oral lipid challenge, which suggests a proinflammatory state in these mice. However, circulating cytokines measured at overnight fasting (Table 8) were indistinguishable in the treated and untreated mice indicating a negligible inflammatory response to the *E. coli* supplementation. Previously, it was shown that an antibiotic treatment improved glucose tolerance of *ob*/*ob* mice and reduced the intestinal TNFα mRNA expression [6]. In the present study, the lack of change on glucose tolerance coincided with a similar amount of intestinal TNFα mRNA expression, supporting the fact that *E. coli* treatment did not exert a bona fide inflammatory response in the non-obese chow fed mice. However, a combination of the *E. coli* treatment and high fat diet feeding could impair the barrier function and lead to proinflammatory responses and insulin resistance in mice.

The most unexpected finding was how the *E. coli* treatment resulted in redistribution of lipids into different pools. During a lipid challenge test, *E. coli* treated mice showed blunted appearance of plasma TG. After the OLTT, cecal lipids were similar in both treated and non-treated groups, suggesting that intestinal lipid malabsorption was unlikely a cause of suppressed plasma TG occurrence. In a post-absorptive state (6h food deprivation in a light cycle), the *E*. *coli* treated mice showed increased plasma TG but this effect disappeared after overnight fast. Based on the data, it is hypothesized that excessive *E. coli* colonization in the gut causes retention of dietary TG in the intestine and delays the release of TG to the circulation. To support this view, mRNA expression of MTTP, a gene involved in intestinal lipoprotein secretion, and plasma TG concentration were both elevated in the treated mice at the post-absorptive state. Recently, Robertson *et al.* showed that lipids can be stored in enterocytes and be released in several hours later in response to a glucose challenge [28]. Also recently been demonstrated by Fielding *et al.,* a part of the lipids originating from a first meal were found in the chylomicrons of the following meal [29]. These data shows that the amount of intestinal E. *coli* has clinical relevance in regulating the kinetics of dietary fats entering the general circulation.

Ectopic fat accumulation is one of hallmark features of insulin resistance and observations of the excessive fat accumulation in the liver and skeletal muscle in insulin resistance individuals and animals supports this argument. However, the intestine as a transient lipid storage pool has not been fully recognized. Recently, Zhu *et al.* applied a coherent anti-Stokes Raman Scattering imaging technique and observed that enterocytes are able to store lipids in droplets after a fat challenge [30]. Subsequently, Uchida *et al.* showed a reduced intestinal TG secretion in response to a dietary fat challenge in obese compared to lean mice [31]. Similarly, Douglass *et al.* reported that *ob*/*ob* mice accumulate TGs in the intestine at the fed state and after an oral lipid challenge [32]. In the present study, treating chow-fed mice with *E*. *coli* isolated from the feces of *ob*/*ob* mice blunted TG appearance in the circulation and increased lipid content measured in the ileum after an oral lipid challenge, reminiscent to the findings in *ob*/*ob* mice published by Douglass *et al.* These data suggest that deregulation of intestinal lipid metabolism in *ob*/*ob* can be mimicked by supplementing mice with the *E. coli* isolated from the feces of *ob*/*ob* mice. On the other hand, other abnormalities resulting from the leptin mutation such as hyperphagia, obesity and insulin resistance measured by oral glucose tolerance test were not affected by the *E. coli* treatment.

Another ectopic lipid pool is the liver and accumulation of fat in the liver links to many pathogenic conditions such as non-alcoholic and alcoholic fatty liver disease as well as non-alcoholic steatohepatitis. Dysbiosis in the gut microbiota has been hypothesized to cause NAFLD and NASH [33]. In the present study, hepatic TG concentrations measured at the post-absorptive state were higher in the *E. coli* treated mice than the untreated mice. Gene expression analysis and plasma ketone bodies measurements indicated a compensatory increase of fatty acid oxidation in the treated mice, a phenomenon commonly found in NAFLD [34]. The data imply a causal relationship between the intestinal *E. coli* and the development of NAFLD. Interestingly, both liver fat and circulation TG in an overnight fast state were unaffected by the *E. coli* treatment.

In summary, this example demonstrates that residential *E. coli* plays a significant role in regulating host lipid metabolism by changing the distribution of TG in the intestine, liver and plasma. However, body weight and oral glucose tolerance of mice were unaffected by the *E. coli* supplementation. Altogether, the data extend the knowledge of residential *E. coli* beyond the pathogenesis of infectious diseases to its role in homeostatic regulation of dietary lipids.

### Example 2:

In the small intestine, dietary lipids are digested before being absorbed by the enterocytes. Once the dietary lipids are inside of cells, they go through several synthesis and transportation steps before being released to the lymphatic circulation via chylomicron. To determine the impacts of the E. coli treatment on lipid metabolism in the intestine of the mice, we analyzed the expression of genes and proteins that are important for the lipid synthesis and transportation pathways. First, we examined the expression of fatty acid transporter protein 4 (FATP4), the main transporter mediating the absorption of dietary lipids. Our results showed that the mRNA expressions of fatp4 were not affected by the E. coli treatment or by the oral lipid tolerance test. However, the FATP4 protein levels did not show the same expression pattern as the fatp4 mRNA. In the control mice, the oral lipid challenge increased FATP4 protein levels by 2 fold, whereas in the E. coli treated mice, FATP4 expression did not change after the lipid challenge. Second, we examined the mRNA and protein expressions of monoacylglyceral acyltransferase (MGAT) in the intestine. In the enterocytes, MGAT is one of enzymes responsible for esterifying fatty acids to form triglyceride. At the mRNA level, the E. coli treatment and the lipid challenge had no impact on mgat expression, a result similar to that of fatp4. At the protein level, MGAT expression was elevated by the lipid challenge in the control mice but not in the E. coli treated mice, a result also similar to that of FATP4. Third, we measured the mRNA and protein of microsomal triglyceride transfer protein (MTTP) in the intestine. MTTP is critical for the synthesis of the triglyceride rich prechylomicron transport vesicles in the endoplasmic reticulum (ER) by fusing the apoB48 phospholipid rich particles to lipid droplets (35). Our results showed that the lipid challenge induced the mRNA expression of mttp in both untreated and E. coli treated mice to a similar extent. Under the same lipid challenge condition, MTTP protein concentrations were elevated in untreated mice but not in the E. coli treated mice. Lastly, we measured the protein levels of SEC24c. SEC24c is found in the prechylomicron transport vesicles and is important for the trafficking of prechylomicron vesicles from ER to Golgi in the intestinal enterocytes (36). In the untreated mice, the SEC24c protein levels were higher after the lipid challenge than before the challenge. In the E. coli treated mice, SEC24c protein concentrations were not affected by the lipid challenge.

In all the genes that we measured, mRNA expressions did not always correspond to the protein levels. The discrepancy between the mRNA and protein concentrations suggests that the presence of E. coli may affect protein synthesis or protein degradation, which results in down regulation of cellular proteins required for lipid synthesis and trafficking. In the enterocytes, ER is an important organelle for protein synthesis/degradation and lipid synthesis/trafficking. Accumulation of unfold proteins in ER causes ER stress, and the ER stress has been shown to affect the lipogenesis and maintenance of lipid metabolism via two ER stress pathways PERK-eiF2α-ATF4 and IRE1α-XBP1 (37). In addition, E. coli has been shown to causes ER stress in the intestine (38,39). Based on the literature evidence, we hypothesized that ER stress is elevated in the E. coli treated mice. Our results showed that the E. coli treatment increased xbpl mRNA levels independent of the oral lipid challenge. The E. coli treatment also increased the mRNA of atf4 and chop when compared to the untreated mice, but the differences were significant in the condition of the lipid challenge.

In conclusion, our data indicate that the E. coli treatment suppressed the dietary lipid-induced expression of proteins that are necessary for the synthesis and trafficking of triglyceride in the intestine. In particular, the lack of up-regulation in MTTP and SEC24c proteins can limit the transportation of prechylomicron vesicles from ER to Golgi, which explains the retention of lipids in the intestine of the E. coli treated mice. Although the molecular mechanisms remain to be identified, elevated ER stress in the intestine may contribute to the dysregulation of intestinal lipid metabolism-caused by the E. coli treatment.

### References

1. IDF. International Diabetes Federation diabetes Atlas. 2012; Available from: http://www.idf.org/diabetesatlas/.
2. Qatanani, M. and M.A. Lazar, Mechanism of obesity-associated Insulin resistance: many choices on the menu. Genes Dev, 2007. 21(12): p. 1443-55.
3. Larsen, N., et al., Gut microbiota in human adults with type 2 diabetes differs from non-diabetic adults. PLoS One, 2010. 5(2): p. e9085.
4. Wu, X., et al., Molecular characterisation of the faecal microbiota in patients with type II diabetes. Curr Microbiol, 2010. 61(1): p. 69-78.
5. Qin, J., et al., A metagenome-wide association study of gut microbiota in type 2 diabetes. Nature, 2012. 490(7418): p. 55-60.
6. Membrez, M., et al., Gut microbiota modulation with norfloxacin and ampicillin enhances glucose tolerance in mice. FASEB J, 2008. 22(7): p. 2416-26.
7. Rabot, S., et al., Germ-free C57BL/6J mice are resistant to high-fat-diet-induced insulin resistance and have altered cholesterol metabolism. FASEB J, 2010. 24(12): p. 4948-59.
8. Cani, P.D., et al., Metabolic endotoxemia initiates obesity and insulin resistance. Diabetes, 2007. 56(7): p. 1761-72.
9. Lassenius, M.I., et al., Bacterial endotoxin activity in human serum is associated with dyslipidemia, Insulin resistance, obesity, and chronic inflammation. Diabetes Care, 2011. 34(8): p. 1809-15.
10. Pussinen, P.J., et al., Endotoxemia is associated with an increased risk of incident diabetes. Diabetes Care, 2011. 34(2): p. 392-7.
11. Harris, K., et al., Is the gut microbiota a new factor contributing to obesity and its metabolic disorders? J Obes, 2012. 2012: p. 879151.
12. Moreira, A.P., et al., Influence of a high-fat diet on gut microbiota, intestinal permeability and metabolic ehdotoxaemia. Br J Nutr, 2012. 108(5): p. 801-9.
13. Amar, J., et al., Energy intake is associated with endotoxemia in apparently healthy men. Am J Clin Nutr, 2008. 87(5): p. 1219-23.
14. Clemente-Postigo, M., et al., Endotoxin increase after fat overload is related to postprandial hypertriglyceridemia in morbidly obese patients. J Lipid Res, 2012. 53(5): p. 973-8.
15. Amar, J., et al., Intestinal mucosal adherence and translocation of commensal bacteria at the early onset of type 2 diabetes: molecular mechanisms and probiotic treatment. EMBO Mol Med, 2011. 3(9): p. 559-72.
16. Alteri, C.J. and H.L. Mobley, Escherichia coli physiology and metabolism dictates adaptation to diverse host microenvironments. Curr Opin Microbiol, 2012. 15(1): p. 3-9.
17. Qadri, F., et al., Enterotoxigenic Escherichia coli in developing countries: epidemiology, microbiology, clinical features, treatment, and prevention. Clin Microbiol Rev, 2005. 18(3): p. 465-83.
18. Schultz, M., Clinical use of E. coli Nissle 1917 in inflammatory bowel disease. Inflamm Bowel Dis, 2008. 14(7): p. 1012-8.
19. Santacruz, A., et al., Gut microbiota composition is associated with body weight, weight gain and biochemical parameters in pregnant women. Br J Nutr, 2010. 104(1): p. 83-92.
20. Carson, C.A., et al., Comparison of ribotyping and repetitive extragenic palindromic-PCR for identification of fecal Escherichia coli from humans and animals. Appl Environ Microbiol, 2003. 69(3): p. 1836-9.
21. Dombek, P.E., et al., Use of repetitive DNA sequences and the PCR To differentiate Escherichia coli isolates from human and animal sources. Appl Environ Microbiol, 2000. 66(6): p. 2572-7.
22. Folch, J., M. Lees, and G.H. Sloane Stanley, A simple method for the isolation and purification of total lipides from animal tissues. J Biol Chem, 1957. 226(1): p. 497-509.
23. Naito, E., et al., Beneficial effect of oral administration of Lactobacillus casei strain Shirota on Insulin resistance in diet-induced obesity mice. J Appl Microbiol, 2011. 110(3): p. 650-7.
24. Abu-Elheiga, L., et al., Continuous fatty acid oxidation and reduced fat storage in mice lacking acetyl-CoA carboxylase 2. Science, 2001. 291(5513): p. 2613-6.
25. Aoyama, T., et al., Altered constitutive expression of fatty acid-metabolizing enzymes in mice lacking the peroxisome proliferator-activated receptor alpha (PPARalpha). J Biol Chem, 1998. 273(10): p. 5678-84.
26. Cani, P.D., et al., Changes in gut microbiota control inflammation in obese mice through a mechanism involving GLP-2-driven improvement of gut permeability. Gut, 2009. 58(8): p. 1091-103.
27. Cani, P.D., et al., Changes in gut microbiota control metabolic endotoxemia-induced inflammation in high-fat diet-induced obesity and diabetes in mice. Diabetes, 2008. 57(6): p. 1470-81.
28. Robertson, M.D., et al., Mobilization of enterocyte fat stores by oral glucose in humans. Gut, 2003. 52(6): p. 834-9.
29. Fielding, B.A., et al., Postprandial lipemia: the origin of an early peak studied by specific dietary fatty acid intake during sequential meals. Am J Clin Nutr, 1996. 63(1): p. 36-41.
30. Zhu, J., et al., A dynamic, cytoplasmic triacylglycerol pool in enterocytes, revealed by ex vivo and in vivo coherent anti-Stokes Raman spattering imaging. J Lipid Res, 2009. 50(6): p. 1080-9.
31. Uchida, A., et al., Reduced triglyceride secretion in response to an acute dietary fat challenge in obese compared to lean mice. Front Physiol, 2012. 3: p. 26.
32. Douglass, J.D., et al., Intestinal mucosal triacylglycerol accumulation secondary to decreased lipid secretion in obese and high fat fed mice. Front Physiol, 2012. 3: p. 25.
33. Abu-Shanab, A. and E.M. Quigley, The role of the gut microbiota in nonalcoholic fatty liver disease. Nat Rev Gastroenterol Hepatol, 2010. 7(12): p. 691-701.
34. Begriche, K., et al., Mitochondrial adaptations and dysfunctions in nonalcoholic fatty liver disease. Hepatology, 2013.
35. Sylvie Demignot, Frauke Beilstein, Etienne Morel. Triglyceride-rich lipoproteins and cytosolic lipid droplets in enterocytes: Key players in intestinal physiology and metabolic disordersq Biochimie 96 (2014) 48-55
36. Xiaoyue Pan and M. Mahmood Hussain. Gut triglyceride production. Biochim Biophys Acta. 2012; 1821(5): 727-735.
37. Xue-Qun Zhang, Cheng-Fu Xu, Chao-Hui Yu, Wei-Xing Chen, You-Ming Li. Role of endoplasmic reticulum stress in the pathogenesis of nonalcoholic fatty liver disease. World J Gastroenterol 2014; 20(7):1768-1776
38. Nathalie Rolhion, Nicolas Barnich, Marie-Agne's Bringer, Anne-Lise Glasser, Julien Ranc, Xavier He'buterne, Paul Hofman, Arlette Darfeuille-Michaud. Abnormally expressed ER stress response chaperone Gp96 in CD favours adherent-invasive Escherichia coli invasion. Gut 2010;59:1355-1362
39. Nathalie Rolhion, Paul Hofman and Arlette Darfeuille-Michaud. The endoplasmic reticulum stress response chaperone Gp96, a host receptor for Crohn disease-associated adherent-invasive Escherichia coli. Gut Microbes 2011;2(2):115-119

Although the foregoing invention has been described in some detail by way of illustration and example for purposes of clarity of understanding, one of skill in the art will appreciate that certain changes and modifications may be practiced within the scope of the appended claims.

## Claims

1. A method for predicting whether a subject is at risk of developing hypertriglyceridemia, the method comprising:
a) transforming a stool sample obtained from the subject to detect the level of Escherichia coli (E. coli) in the sample; and
b) determining that a higher level of E. coli in the sample compared to a control sample predicts that the subject is at risk of developing hypertriglyceridemia.

2. The method of claim 1, wherein detecting the level of E. coli comprises performing nucleic acid amplification.

3. The method of claim 1, wherein detecting the level of E. coli comprises performing colony counting.

4. The method of claim 1, wherein the control sample is from a healthy control subject or a subject that is not at risk of developing hypertriglyceridemia.

5. A method for diagnosing hypertriglyceridemia in a subject, the method comprising:
a) transforming a stool sample obtained from the subject to detect the level of E. coli in the sample; and
b) determining that a higher level of E.coli in the sample compared to a control sample indicates a diagnosis of hypertriglyceridemia in the subject.

6. The method of claim 5, wherein detecting the level of E. coli comprises performing nucleic acid amplification.

7. The method of claim 6, wherein nucleic acid amplification is performed using oligonucleotides whose sequences are set forth in SEQ ID NOs:3 and 4.

8. The method of claim 6, wherein nucleic acid amplification comprises enterobacterial repetitive intergenic consensus PCR or repetitive extragenic PCR.

## Patentansprüche

1. Verfahren zum Vorhersagen, ob ein Subjekt gefährdet ist, Hypertriglyceridämie zu entwickeln, wobei das Verfahren Folgendes umfasst:
a) Transformieren einer Stuhlprobe, die von dem Subjekt erhalten wird, um den Gehalt von Escherichia coli (E. coli) in der Probe zu detektieren; und
b) Bestimmen, dass ein höherer Gehalt von E. coli in der Probe im Vergleich zu einer Kontrollprobe vorhersagt, dass das Subjekt gefährdet ist, Hypertriglyceridämie zu entwickeln.

2. Verfahren nach Anspruch 1, wobei das Detektieren des Gehalts von E. coli das Ausführen einer Nukleinsäureamplifikation umfasst.

3. Verfahren nach Anspruch 1, wobei das Detektieren des Gehalts von E. coli das Ausführen einer Keimzahlbestimmung umfasst.

4. Verfahren nach Anspruch 1, wobei die Kontrollprobe von einem gesunden Kontrollsubjekt oder einem Subjekt stammt, das nicht gefährdet ist, Hypertriglyceridämie zu entwickeln.

5. Verfahren zum Diagnostizieren von Hypertriglyceridämie in einem Subjekt, wobei das Verfahren Folgendes umfasst:
a) Transformieren einer Stuhlprobe, die von dem Subjekt erhalten wird, um den Gehalt von E. coli in der Probe zu detektieren; und
b) Bestimmen, dass ein höherer Gehalt von E. coli in der Probe im Vergleich zu einer Kontrollprobe eine Diagnose von Hypertriglyceridämie in dem Subjekt anzeigt.

6. Verfahren nach Anspruch 5, wobei das Detektieren des Gehalts von E. coli das Ausführen einer Nukleinsäureamplifikation umfasst.

7. Verfahren nach Anspruch 6, wobei die Nukleinsäureamplifikation unter Verwendung von Oligonukleotiden ausgeführt wird, deren Sequenzen in den SEQ ID-Nr.:3 und 4 dargelegt sind.

8. Verfahren nach Anspruch 6, wobei die Nukleinsäureamplifikation eine enterobakterielle repetitive intragenische Consensus-PCR oder eine repetitive extragenische PCR umfasst.

## Revendications

1. Procédé permettant de prédire si un sujet est à risque de développer une hypertriglycéridémie, le procédé comprenant :
a) la transformation d'un échantillon de selles provenant d'un sujet afin de détecter le niveau d'Escherichia coli (E. coli) dans l'échantillon ; et
b) la détermination qu'un niveau plus élevé d'E. coli dans l'échantillon comparativement à un échantillon témoin prédit que le sujet est à risque de développer une hypertriglycéridémie.

2. Procédé selon la revendication 1, dans lequel la détection du niveau d'E. coli comprend la réalisation d'une amplification d'acide nucléique.

3. Procédé selon la revendication 1, dans lequel la détection du niveau d'E. coli comprend la réalisation du comptage des colonies.

4. Procédé selon la revendication 1, dans lequel l'échantillon témoin provient d'un sujet témoin sain ou d'un sujet qui n'est pas à risque de développer une hypertriglycéridémie.

5. Procédé de diagnostic de l'hypertriglycéridémie chez un sujet, le procédé comprenant :
a) la transformation d'un échantillon de selles provenant d'un sujet afin de détecter le niveau d'Escherichia coli (E. coli) dans l'échantillon ; et
b) la détermination qu'un niveau plus élevé d'E. coli dans l'échantillon comparativement à un échantillon témoin indique un diagnostic d'hypertriglycéridémie chez le sujet.

6. Procédé selon la revendication 5, dans lequel la détection du niveau d'E. coli comprend la réalisation d'une amplification d'acide nucléique.

7. Procédé selon la revendication 6, dans lequel l'amplification d'acide nucléique est réalisée au moyen d'oligonucléotides dont les séquences sont représentées dans les SEQ ID n° : 3 et 4.

8. Procédé selon la revendication 6, dans lequel l'amplification d'acide nucléique comprend une PCR consensus répétitive intergénique ou une PCR répétitive extragénique entérobactérienne.
